# DEMANDE DE BREVET EUROPEEN

(11) **EP 2 153 817 A1**
(43) Date de publication de la demande: **17.02.2010**
(21) Numéro de dépôt: 09165250.3
(22) Date de dépôt: 10.07.2009
(51) Int. Cl.: A61K 8/895, A61K 8/58, A61K 8/35, A61K 8/49, A61Q 17/04

(54) **Kit de protection solaire**

(30) Priorité: 10.07.2008 FR 0854723
(71) Demandeur: L'Oréal, 75008 Paris (FR)
(72) Inventeur: Gaudry, Anne-Laure, 77320, La Ferte Gaucher (FR); Josso, Martin, 75007, Paris (FR); Boschet, Cécile, 94240, L'Haye-Les-Roses (FR)
(74) Mandataire: Le Coupanec, Pascale A.M.P.

(57) **Abrégé**

La présente invention concerne un kit de photoprotection des matières kératiniques contre un rayonnement UV dans la gamme au dessus de 280 nm couvrant les UV A et les UV B, comprenant au moins deux compositions différentes conditionnées séparément, ledit kit comprenant au moins un composé (X), au moins un composé (Y), et au moins un système filtrant hydrophobe comprenant (i) au moins un filtre solaire organique hydrophobe (A) capable d'absorber les rayonnements UV de 320 à 400 nm et au moins un filtre solaire organique hydrophobe (B) capable d'absorber les rayonnements UV de 280 à 320 nm, et éventuellement un filtre minéral (D) ; (ii) au moins un filtre solaire organique hydrophobe (C) capable d'absorber simultanément les rayonnements UV de 280 à 320 nm et 320 à 400 nm, et éventuellement un filtre minéral (D) ; ou (iii) au moins un filtre solaire organique hydrophobe (A) capable d'absorber les rayonnements UV de 320 à 400 nm et au moins soit un filtre solaire organique hydrophobe (C) capable d'absorber simultanément les rayonnements UV de 280 à 320 nm et 320 à 400 nm, soit un filtre minéral (D) ;
l'un au moins desdits composés (X) et (Y) étant siliconé.

## Description

La présente invention concerne le domaine de la protection solaire. Elle concerne plus particulièrement un kit de protection des matières kératiniques, en particulier de la peau, contre les rayonnements UV-A et UV-B comprenant au moins deux compositions et au moins deux composés (X) et (Y), aptes à réagir ensemble, l'un au moins des composés étant siliconé ainsi qu'au moins un système filtrant hydrophobe comprenant :
(i) soit au moins un filtre solaire organique UVA hydrophobe et au moins un filtre solaire organique hydrophobe UVB et éventuellement un filtre minéral ;
(ii) soit au moins un filtre solaire organique hydrophobe UVA et UVB et éventuellement un filtre minéral ;
(iii) soit au moins un filtre solaire organique UVA hydrophobe et au moins un filtre minéral, ou au moins un filtre organique hydrophobe UVA et UVB.

L'invention concerne également un procédé de photoprotection des matières kératiniques contre un rayonnement UV-A et UV-B, ainsi qu'un film de photoprotection susceptible d'être obtenu par un tel procédé.

Il est bien connu que les rayons UV-A, de longueurs d'onde comprises entre 320 et 400 nm, provoquant le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. D'autre part, on sait que les radiations lumineuses de longueurs d'onde comprises entre 280 et 320 nm, connus sous la dénomination d'UV-B, provoquent des érythèmes et des brûlures cutanées qui peuvent nuire au développement du bronzage naturel.

Il est donc souhaitable de filtrer aussi bien les rayonnements UV-A que les rayonnements UV-B.

De manière générale, les compositions cosmétiques destinées à la photoprotection de la peau contiennent des filtres UV organiques et/ou inorganiques qui fonctionnent selon leur nature chimique et leurs propriétés physiques, par absorption, réflexion ou diffusion du rayonnement UV.

L'objectif dans la mise au point de compositions solaires comprenant de tels filtres solaires est généralement d'obtenir le meilleur rapport taux de filtres solaires/efficacité.

L'efficacité d'une composition solaire pour la peau se traduit habituellement en terme de facteur de protection solaire (abrégé SPF) qui est défini par le rapport de la quantité d'énergie nécessaire pour provoquer un début d'érythème sur la peau protégée par l'agent filtrant les radiations UV sur la quantité d'énergie nécessaire pour provoquer un début d'érythème sur la peau non protégée. Pour atteindre des niveaux d'efficacité significatifs, l'homme de l'art est couramment amené à introduire les agents photoprotecteur en quantité importante et ce d'autant plus que le niveau d'efficacité requis est élevé.

A cet effet, il est couramment fait usage de différents types de filtres existant sur le marché : les filtres minéraux et les filtres chimiques (ou filtres solaires organiques).

Ces produits solaires existent notamment sous forme de lait, de crème, de spray, de stick, de lingettes imprégnées. Ces produits peuvent permettre d'atteindre des niveaux de photoprotection très élevés.

Toutefois, lorsqu'ils sont appliqués sur la peau, ces produits peuvent avoir tendance à s'enlever du fait de différents facteurs tel que la transpiration, un bain de mer, un frottement mécanique comme, par exemple, le contact avec une serviette ou du sable.

Il existe, par conséquent, sur le marché des produits solaires un besoin de disposer de produits cosmétiques et/ou de soin dotés de qualités de protections solaires efficaces des matières kératiniques à l'encontre des rayonnements UV-A et UV-B, bénéficiant d'un SFP élevé tout en résistant aux agressions extérieurs notamment telles que mentionnées ci-dessus. En particulier, les consommateurs sont en attente de produits de protection solaire confortables, présentant une innocuité vis-à-vis des matières kératiniques tout en assurant une forte protection solaire.

Dans certain cas, l'utilisateur peut même souhaiter obtenir une protection solaire quasi-totale et surtout avoir l'assurance qu'elle va résister aux agressions. Typiquement, l'utilisateur peut désirer protéger une cicatrice, à savoir une zone de la peau localisée, après une intervention chirurgicale.

Un utilisateur à la recherche d'une telle protection quasi-totale localisée peut actuellement recourir à un pansement opaque. Toutefois, une telle solution peut ne pas paraître satisfaisante, en particulier sur le plan esthétique.

Le document GB 2 407 496 enseigne la possibilité de réaliser des films siliconés, qui adhèrent à la peau, servant de barrière, à usage cosmétique, voire pour diffuser des substances pharmaceutiques. Ces films peuvent notamment comprendre des filtres solaires.

Le document EP 973 493 concerne des matières sol/gel dopées d'écran solaire utiles pour la protection de tissus corporels contre un rayonnement solaire dans lesquelles l'écran solaire dopé s'avère être des molécules d'écran solaire chimique capables d'absorber un rayonnement UV dans la gamme au-dessus de 250 nm et dans lesquelles lesdites molécules d'écran solaire sont physiquement piégées.

Quant au document WO 2007/071706, il concerne un procédé de maquillage ou de soin des matières kératiniques comprenant l'application de composés, l'un au moins étant siliconés à l'aide d'une composition de soin contenant en outre des produits solaires.

De manière inattendue, les inventeurs ont découvert que les inconvénients précités des compositions actuellement sur le marché pouvaient être résolus par la mise en oeuvre d'un kit dédié à la formation d'un film particulier comprenant des filtres solaires contre les rayonnements UV-A et UV-B spécifiques. Les inventeurs ont, en effet constaté, de façon surprenante que ledit film obtenu à l'aide dudit kit offre une protection solaire significativement améliorée.

Contre toute attente, les inventeurs ont donc découvert qu'il était possible de disposer de films photoprotecteurs à efficacité accrue contre les UV-A et les UV-B en utilisant des quantités moindres de filtres solaires par rapport à un produit solaire classique.

Les inventeurs ont en particulier mis en évidence qu'en associant des filtres contre les UV A et les UV B en mélange ou mixtes, au sein du kit selon la présente invention, il est possible d'atteindre des valeurs de SPF bien supérieures à ceux obtenus quand seuls les filtres UV B sont utilisés.

Les inventeurs ont également mis en évidence qu'en associant au moins un filtre minéral avec au moins un filtre organique UVA hydrophobe, au sein du kit selon la présente invention, il est possible d'atteindre des valeurs de SPF bien supérieures à celles obtenues quand le filtre minéral et le filtre organique UVA hydrophobe sont utilisés seuls.

Ainsi, conformément à l'un des objets de la présente invention, il est maintenant proposé un kit de photoprotection des matières kératiniques contre un rayonnement UV dans la gamme au dessus de 280 nm couvrant les UVA et les UVB, comprenant au moins deux compositions différentes conditionnées séparément, ledit kit comprenant :
- au moins un composé (X),
- au moins un composé (Y),
- éventuellement au moins un catalyseur ou au moins un peroxyde, et
- au moins un système filtrant hydrophobe comprenant :
   (i) au moins un filtre solaire organique hydrophobe (A) capable d'absorber les rayonnements UV de 320 à 400 nm et au moins un filtre solaire organique hydrophobe (B) capable d'absorber les rayonnements UV de 280 à 320 nm et/ou au moins un filtre minéral (D) ;
   (ii) au moins un filtre solaire organique hydrophobe (C) capable d'absorber simultanément les rayonnements UV de 280 à 320 nm et 320 à 400 nm, et éventuellement un filtre minéral (D) ; ou
   (iii) au moins un filtre solaire organique hydrophobe (A) capable d'absorber les rayonnements UV de 320 à 400 nm et au moins soit un filtre solaire organique hydrophobe (C) capable d'absorber simultanément les rayonnements UV de 280 à 320 nm et 320 à 400 nm, soit un filtre minéral (D) ;
l'un au moins desdits composés (X) et (Y) étant siliconés et à la condition que les composés (X), (Y) et éventuellement le catalyseur ou le peroxyde ne soient pas présents simultanément dans la même composition, lesdits composés (X) et (Y) étant susceptibles de réagir ensemble par une réaction d'hydrosilylation en présence dudit catalyseur ou par une réaction de condensation ou par une réaction de réticulation en présence dudit peroxyde lorsqu'ils sont mis en contact les uns avec les autres.

Selon un mode de réalisation, le kit comprend au moins deux compositions différentes conditionnées séparément, ledit kit comprenant au moins :
- une première composition contenant, dans un milieu physiologiquement acceptable, au moins un composé (X), et
- une seconde composition contenant, dans un milieu physiologiquement acceptable, au moins un composé (Y),
ledit éventuel catalyseur ou ledit éventuel peroxyde étant présent dans l'une ou l'autre desdites première et seconde compositions ou présent éventuellement dans une troisième composition et lesdits filtres solaires (A), (B), (C), et/ou (D), étant présents dans l'une ou l'autre desdites première et seconde compositions ou présent éventuellement dans ladite troisième composition.

Un autre objet de l'invention est un procédé de photoprotection des matières kératiniques contre un rayonnement UV dans la gamme au dessus de 280 nm couvrant les régions UV-A et UV-B, comprenant l'application sur lesdites matières :
a) d'au moins un composé (X),
b) d'au moins un composé (Y), et
c) d'au moins un système filtrant hydrophobe comprenant :
   (i) au moins un filtre solaire organique hydrophobe (A) capable d'absorber les rayonnements UV de 320 à 400 nm et au moins un filtre solaire organique hydrophobe (B) capable d'absorber les rayonnements UV de 280 à 320 nm et/ou au moins un filtre minéral (D) ;
   (ii) au moins un filtre solaire organique hydrophobe (C) capable d'absorber simultanément les rayonnements UV de 280 à 320 nm et 320 à 400 nm, et éventuellement un filtre minéral (D) ; ou
   (iii) au moins un filtre solaire organique hydrophobe (A) capable d'absorber les rayonnements UV de 320 à 400 nm et au moins soit un filtre solaire organique hydrophobe (C) capable d'absorber simultanément les rayonnements UV de 280 à 320 nm et 320 à 400 nm, soit un filtre minéral (D) ;
l'un au moins desdits composés (X) et (Y) étant siliconés, lesdits composés (X) et (Y) étant susceptibles de réagir ensemble, ladite réaction étant une réaction d'hydrosilylation en présence d'au moins un catalyseur, une réaction de condensation ou une réaction de réticulation en présence d'au moins un peroxyde à la condition que les applications a), b) et c) aient lieu soit (i) simultanément par mélange extemporané préalable, soit (ii) par mélange au moment de leur application de façon simultanée ou séquencée selon un ordre indifférent sous réserve que les conditions dudit mélange soit propice à la réaction entre lesdits composés (X) et (Y).

Enfin, un autre objet de l'invention est un film de photoprotection susceptible d'être obtenu par le procédé de photoprotection des matières kératiniques telles que définies précédemment.

Le kit de photoprotection selon l'invention présente notamment l'avantage de permettre l'obtention de films phototoprotecteurs aptes à protéger des zones ciblées de la peau, par exemple des lésions, des cicatrices, des blessures tout en présentant une bonne adhérence sur les matières kératiniques et pouvant être retiré facilement après usage.

Les films photoprotecteurs ainsi obtenus grâce aux kits selon l'invention s'avèrent former un film résiduel homogène, non brillant, non collant et confortable et permettent un maquillage ultérieur des zones protégées. De plus, les films obtenus à l'issue de la réaction entre les composés (X) et (Y) peuvent, selon un aspect particulier de l'invention, présenter une matrice relativement transparente ou translucide procurant ainsi des qualités esthétiques particulièrement recherchées.

D'autre part, les inventeurs ont également pu observer une stabilité de certaines associations de filtres solaires dans lesdits films photoprotecteurs qui habituellement mis en contact dans une même crème solaire interagissent défavorablement les uns avec les autres, altérant les qualités de protection de ces mêmes compositions. Il est ainsi rendu possible des associations de filtres jusqu'alors proscrites dans le domaine de la protection solaire.

Dans le cadre de la présente invention, par « transparent ou translucide », on entend la faculté à laisser passer la lumière sans provoquer de déviation par réfraction ou réflexion.

Plus particulièrement, on entend par « transparence ou translucidité », la faculté à transmettre en moyenne au moins 25 % de la lumière de la fenêtre de longueurs d'onde de 400 à 700 nm, de préférence 50 % de la lumière à travers un film conforme à l'invention par exemple d'épaisseur dix microns.

De par sa présence dans les conditions requises selon l'invention, le produit de réaction des composés (X) et (Y) assure une cohésion satisfaisante du film rendant ainsi possible une photoprotection efficace. Le film photoprotecteur présente, en outre, une bonne rémanence à l'eau, à la transpiration et au lavage, ainsi qu'une bonne tenue ou persistance dans le temps.

Ces films sont par ailleurs facilement retirable ou décollable après usage par frottement, par exemple provoquant le peluchage du film, ou à la manière d'un sparadrap.

Selon un autre aspect, l'invention concerne une composition cosmétique de photoprotection des matières kératiniques, en particulier la peau, contre un rayonnement UV dans la gamme au dessus de 280 nm couvrant les UV A et les UV B comprenant, dans un milieu physiologiquement acceptable :
- au moins un composé (X),
- au moins un composé (Y),
- au moins un catalyseur, et
- au moins un système filtrant hydrophobe comprenant :
   (i) au moins un filtre solaire organique hydrophobe (A) capable d'absorber les rayonnements UV de 320 à 400 nm et au moins un filtre solaire organique hydrophobe (B) capable d'absorber les rayonnements UV de 280 à 320 nm et/ou au moins un filtre minéral (D) ;
   (ii) au moins un filtre solaire organique hydrophobe (C) capable d'absorber simultanément les rayonnements UV de 280 à 320 nm et 320 à 400 nm, et éventuellement un filtre minéral (D) ; ou
   (iii) au moins un filtre solaire organique hydrophobe (A) capable d'absorber les rayonnements UV de 320 à 400 nm et au moins soit un filtre solaire organique hydrophobe (C) capable d'absorber simultanément les rayonnements UV de 280 à 320 nm et 320 à 400 nm, soit un filtre minéral (D) ;
avec au moins un des composés (X) ou (Y) étant un polyorganosiloxane, et lesdits composés (X) et (Y) étant susceptibles de réagir ensemble par une réaction d'hydrosilylation en présence dudit catalyseur, avec au moins un composé parmi les composés (X) et (Y) étant présent dans ladite composition sous une forme encapsulée, ledit catalyseur étant associé à au moins l'un desdits composés (X) ou (Y) encapsulé.

Cette composition peut être comprise dans un kit de photoprotection conforme à la présente invention.

Selon un autre aspect, l'invention concerne un procédé de photoprotection des matières kératiniques, en particulier la peau, contre un rayonnement UV dans la gamme au-dessus de 280 nm couvrant les régions UVA et UVB, comprenant l'application sur lesdites matières kératiniques de ladite composition objet de l'invention, ledit composé (X) et/ou ledit composé (Y) étant présents dans ladite composition sous une forme encapsulée, ruptible à l'application sur lesdites matières kératiniques.

### COMPOSES (X) et (Y)

Le kit selon l'invention comprend au moins un composé (X) et au moins un composé (Y), avec au moins un des composés (X) et (Y) étant siliconé, susceptibles de réagir par réaction d'hydrosilylation en présence d'un catalyseur, de condensation ou par réaction de réticulation en présence d'un peroxyde, tels que définis ci-après. De façon avantageuse, les composés (X) et (Y) réagissent par réaction d'hydrosilylation en présence d'un catalyseur de façon à former un film.

### 1- Composés (X) et (Y) susceptibles de réagir par réaction d'hydrosilylation en présence d'un catalyseur

La réaction d'hydrosilylation a lieu entre au moins un composé (X), au moins un composé (Y) et au moins un catalyseur.

Les composés (X) et (Y) peuvent être présents respectivement au sein du kit selon l'invention dans une première composition et dans une seconde composition, voire dans une autre composition que lesdites première et seconde compositions.

Selon un mode de réalisation particulier, le composé (X) est présent dans une composition (A1) et le composé (Y) est présent dans une composition (A2).

Selon un autre mode de réalisation, les composés (X) et (Y) distincts l'un de l'autre sont présents dans une composition (A3).

La réaction entre les composés (X) et (Y) susceptibles de réagir par hydrosilylation en présence d'un catalyseur, peut être schématisée comme suit de manière simplifiée: 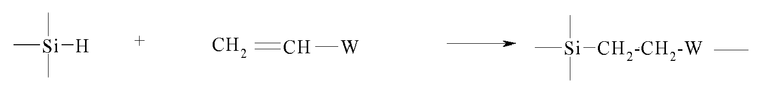 avec W représentant une chaîne carbonée et/ou siliconée contenant un ou plusieurs groupements aliphatiques insaturés.

Dans ce cas, le composé (X) peut être choisi parmi les composés siliconés comprenant au moins deux groupements aliphatiques insaturés. A titre d'exemple, le composé (X) peut être un polyorganosiloxane comprenant une chaîne principale siliconée dont les groupements aliphatiques insaturés sont pendants à la chaîne principale (groupe latéral) ou situés aux extrémités de la chaîne principale du composé (groupe terminal). Dans la suite de la description, on appellera ces composés particuliers des polyorganosiloxanes à groupements aliphatiques insaturés.

Selon un mode de réalisation, le composé (X) et/ou le composé (Y) est porteur d'au moins un groupe polaire, tel que décrit ci-dessous, susceptible de former au moins une liaison hydrogène avec les matières kératiniques. Ce groupe polaire est avantageusement porté par le composé (X) qui comprend au moins deux groupements aliphatiques insaturés.

### Groupes polaires

Selon un mode de réalisation particulier, l'un au moins des composé (X) et (Y), par exemple le composé (X), est porteur d'au moins un groupe polaire susceptible de former au moins une liaison hydrogène avec les matières kératiniques.

Par groupe polaire, on entend un groupe comportant des atomes de carbone et d'hydrogène dans sa structure chimique et au moins un hétéroatome (tel que O, N, S et P), tel que ledit groupe est apte à établir au moins une liaison hydrogène avec les matières kératiniques.

Des composés porteurs d'au moins un groupement apte à établir une liaison hydrogène sont particulièrement avantageux, car ils apportent aux compositions les contenant une meilleure adhérence sur les matières kératiniques.

Le ou les groupe(s) polaire(s) porté(s) par au moins l'un des composés (X) et (Y) est/sont apte(s) à établir une liaison hydrogène, et comporte(nt) soit un atome d'hydrogène lié à un atome électronégatif, soit un atome électronégatif comme par exemple l'atome d'oxygène, d'azote ou de soufre. Lorsque le groupe comporte un atome d'hydrogène lié à un atome électronégatif, l'atome d'hydrogène peut interagir avec un autre atome électronégatif porté, par exemple par une autre molécule, telle que la kératine, pour former une liaison hydrogène. Lorsque le groupement comporte un atome électronégatif, l'atome électronégatif peut interagir avec un atome d'hydrogène lié à un atome électronégatif porté, par exemple par une autre molécule, telle que la kératine, pour former une liaison hydrogène.

Avantageusement, ces groupes polaires peuvent être choisis parmi les groupes suivants :
- acides carboxyliques -COOH,
- alcools, tels que : -CH₂OH ou -CH(R)OH, R étant un radial alkyle comprenant de 1 à 6 atomes de carbone,
- amino de formule -NR₁R₂, dans laquelle les R₁ et R₂ identiques ou différents représentent un radial alkyle comprenant de 1 à 6 atomes de carbone ou l'un des R₁ ou R₂ désigne un atome d'hydrogène, et l'autre des R₁ et R₂ représente un radical alkyle comprenant de 1 à 6 atomes de carbone,
- pyridino,
- amido de formule -NH-COR' ou -CO-NH-R' dans laquelle R' représente un atome d'hydrogène ou un radial alkyle comprenant de 1 à 6 atomes de carbone,
- pyrrolidino choisi de préférence parmi les groupes de formule : 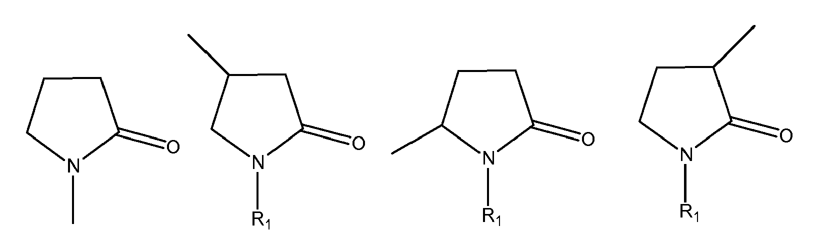 R₁ étant un radial alkyle comprenant de 1 à 6 atomes de carbone,
- carbamoyle de formule -O-CO-NH-R' ou -NH-CO-OR', R' étant tel que défini ci-dessus,
- thiocarbamoyle tel que -O-CS-NH- R' ou -NH-CS-OR', R' étant tel que défini ci-dessus,
- uréyle tel que -NR'-CO-N(R')₂, les groupes R' identiques ou différents étant tels que définis ci-dessus,
- sulfonamido tel que -NR'-S(=O)₂-R', R' répondant à la définition ci-dessus.

De préférence, ces groupes polaires sont présents à une teneur inférieure ou égale à 10 % en poids par rapport au poids de chaque composé (X) ou (Y), de préférence inférieure ou égale à 5 % en poids, par exemple en une teneur allant de 1 à 3 % en poids.

Le ou les groupes polaires peu(ven)t être situé(s) dans la chaîne principale du composé (X) et/ou (Y) ou peuvent être pendants à la chaîne principale ou situés aux extrémités de la chaîne principale du composé (X) et/ou (Y).

Selon un mode de réalisation, le composé (X) est choisi parmi les polyorganosiloxanes comprenant au moins deux groupements aliphatique insaturés, par exemple deux ou trois groupements vinyliques ou allyliques, liés chacun à un atome de silicium.

Selon un mode de réalisation avantageux, le composé (X) est choisi parmi les polyorganosiloxanes comprenant des unités siloxanes de formule (I) suivante: 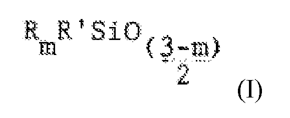 dans laquelle :
- R représente un groupe hydrocarboné monovalent, linéaire ou cyclique, comprenant de 1 à 30 atomes de carbone, de préférence de 1 à 20, et mieux de 1 à 10 atomes de carbone, comme par exemple un radical alkyle à chaîne courte, comprenant par exemple de 1 à 10 atomes de carbone, en particulier un radical méthyle ou encore un groupement phényle, de préférence un radical méthyle,
- m est égal à 1 ou 2 et
- R' représente :
   ○ un groupement hydrocarboné aliphatique insaturé comprenant de 2 à 10, de préférence de 3 à 5 atomes de carbone comme par exemple un groupe vinyle ou un groupe -R"-CH=CHR"' dans lequel R" est une chaîne hydrocarbonée aliphatique divalente, comprenant de 1 à 8 atomes de carbone, liée à l'atome de silicium et R"' est un atome d'hydrogène ou un radical alkyle comprenant de 1 à 4 atomes de carbone, de préférence un atome d'hydrogène. On peut citer comme groupement R' les groupements vinyle, allyle et leurs mélanges ; ou
   ○ un groupement hydrocarboné cyclique insaturé comprenant de 5 à 8 atomes de carbone comme par exemple un groupe cyclohexenyle.

De préférence, R' est un groupement hydrocarboné aliphatique insaturé, de préférence un groupe vinyle.

Selon un mode de réalisation, R représente un radical alkyle comprenant de 1 à 10 atomes de carbone ou encore un groupement phényle, et de préférence un radical méthyle, et R' est un groupe vinyle.

Selon un mode de réalisation particulier, le polyorganosiloxane comprend également des unités de formule (II): 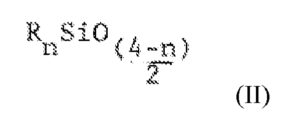 dans laquelle R est un groupe tel que défini plus haut, et n est égal à 1, 2 ou 3.

Selon une variante, le composé (X) peut être une résine de silicone comprenant au moins deux insaturations éthyléniques, ladite résine étant apte à réagir avec le composé (Y). On peut citer par exemple les résines de type MQ ou MT portant elle-même des extrémités réactives insaturées -CH=CH₂.

Ces résines sont des polymères d'organosiloxanes réticulés.

La nomenclature des résines de silicone est connue sous le nom de « MDTQ », la résine étant décrite en fonction des différentes unités monomèriques siloxane qu'elle comprend, chacune des lettres « MDTQ » caractérisant un type d'unité :
• La lettre M représente l'unité monofonctionelle de formule (CH₃)₃SiO_{1/2}, l'atome de silicium étant relié à un seul atome d'oxygène dans le polymère comprenant cette unité ;
• La lettre D signifie une unité difonctionnelle (CH₃)₂SiO_{2/2} dans laquelle l'atome de silicium est relié à deux atomes d'oxygène ;
• La lettre T représente une unité trifonctionnelle de formule (CH₃)SiO_{3/2} dans laquelle l'atome de silicium est relié à trois atomes d'oxygène ;
   Dans les motifs M, D, T définis précédemment, au moins un des groupes méthyles peut être substitués par un groupe R différent du groupe méthyle tel qu'un radical hydrocarboné (notamment alkyle) ayant de 2 à 10 atomes de carbone ou un groupe phényl ou bien encore un groupe hydroxyle.
• La lettre Q signifie une unité tetrafonctionnelle SiO_{4/2} dans laquelle l'atome de silicium est relié à quatre atomes d'hydrogène eux mêmes liés au reste du polymère. Comme exemples de telles résines, on peut citer les résines de silicone MT telles que les poly(phényl-vinylsilsesquioxane) comme celle commercialisées sous la référence SST-3PV1 par la société Gelest.

De préférence, les composés (X) comprennent de 0,01 à 1 % en poids de groupes aliphatiques insaturés.

Avantageusement, le composé (X) est choisi parmi les polyorganopolysiloxanes, notamment ceux comprenant les unités siloxanes (I) et éventuellement (II) décrites précédemment.

Le composé (Y) comprend de préférence au moins deux groupes Si-H (groupes hydrogénosilanes) libres.

Le composé (Y) peut être avantageusement choisi parmi les polyorganosiloxanes comprenant au moins une unité alkylhydrogènosiloxane de formule (III) suivante : 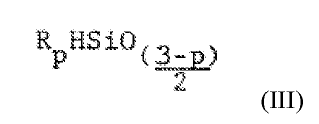 dans laquelle :
- R représente un groupe hydrocarboné monovalent, linéaire ou cyclique, comprenant de 1 à 30 atomes de carbone, comme par exemple un radical alkyle ayant de 1 à 30 atomes de carbone, de préférence de 1 à 20 et mieux de 1 à 10 atomes de carbone, en particulier un radical méthyle, ou encore un groupement phényle et p est égal à 1 ou 2. De préférence R est un groupement hydrocarboné, de préférence le méthyle.

Ces composés (Y) polyorganosiloxanes à unités alkylhydrogènosiloxanes peuvent comprendre en outre des unités de formule (II) telles que définies plus haut.

Le composé (Y) peut être une résine de silicone comprenant au moins un motif choisi parmi les motifs M, D, T, Q tels que définis ci-dessus et comprenant au moins un groupe Si-H tel que les poly(méthyl-hydridosilsesquioxane) commercialisées sous la référence SST-3MH1.1 par la société Gelest.

De préférence, ces composés (Y) polyorganosiloxanes comprennent de 0,5 à 2,5 % en poids de groupes Si-H.

Avantageusement, les radicaux R représentent un groupement méthyle dans les formules (I), (II), (III) ci-dessus.

De préférence, ces polyorganosiloxanes (Y) comprennent des groupes terminaux de formule (CH₃)₃SiO_{1/2}.

Avantageusement, les polyorganosiloxanes (Y) comprennent au moins deux unités alkylhydrogènosiloxane de formule -(H₃C)(H)SiO- et comprennent éventuellement des unités -(H₃C)₂SiO-.

De tels composés polyorganosiloxanes (Y) à groupements hydrogénosilane sont décrits, par exemple, dans le document EP 465 744.

Selon une variante, le composé (X) est choisi parmi les oligomères ou polymères organiques (par organique, on entend des composés dont la chaîne principale est non siliconée, de préférence des composés ne comprenant pas d'atomes de silicium) ou parmi les polymères ou oligomères hybrides organique/silicone, lesdits oligomères ou polymères portant au moins 2 groupements aliphatiques insaturés réactifs, le composé (Y) étant choisi parmi les polyorganosiloxanes (Y) à groupements hydrogénosilane cités ci-dessus.

Selon un mode de réalisation, les composés (X) organiques ou hybrides organique/silicone portant au moins 2 groupements aliphatiques insaturés réactifs, portent au moins un groupe polaire tel que décrit plus haut.

Le composé (X), de nature organique, peut alors être choisi parmi les polymères ou oligomères vinyliques, (méth)acryliques, les polyesters, les polyuréthanes et/ou les polyurées, les polyéthers, les perfluoropolyéthers, les polyoléfines telles que le polybutène, le polyisobutylène, les dendrimères ou les polymères hyper-ramifiés organiques, ou leurs mélanges.

En particulier, le polymère organique ou la partie organique du polymère hybride peut être choisi parmi les polymères suivants :

### a) les polyesters à insaturation(s) éthylénique(s) :

Il s'agit d'un groupe de polymères de type polyester présentant au moins 2 doubles liaisons éthyléniques, réparties de manière aléatoire dans la chaîne principale du polymère. Ces polyesters insaturés sont obtenus par polycondensation d'un mélange :
- de diacides carboxyliques aliphatiques, linéaires ou ramifiés, ou cycloaliphatiques comportant notamment de 3 à 50 atomes de carbone, de préférence de 3 à 20 et mieux de 3 à 10 atomes de carbone, tels que l'acide adipique ou l'acide sébacique, de diacides carboxyliques aromatiques ayant notamment de 8 à 50 atomes de carbone, de préférence de 8 à 20 et mieux de 8 à 14 atomes de carbone, tels que les acides phtaliques, notamment l'acide téréphtalique, et/ou de diacides carboxyliques issus de dimères d'acides gras à insaturations éthyléniques tels que les dimères des acides oléique ou linoléique décrits dans la demande EP 959 066 (paragraphe [0021]) commercialisés sous les dénominations Pripol^{®} par la société Unichema ou Empol^{®} par la société Henkel, tous ces diacides devant être exempts de doubles liaisons éthyléniques polymérisables,
- de diols aliphatiques, linéaires ou ramifiés, ou cycloaliphatiques comportant notamment de 2 à 50 atomes de carbone, de préférence de 2 à 20 et mieux de 2 à 10 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 et mieux de 6 à 15 atomes de carbone tel que le bisphénol A et le bisphénol B, et/ou de dimères diols issus de la réduction des dimères d'acides gras tels que définis précédemment, et
- d'au moins un diacide carboxylique ou son anhydride comportant deux doubles liaisons ou au moins deux diacides carboxyliques ou anhydrides comportant chacun au moins une double liaison éthylénique polymérisable et ayant de 3 à 50 atomes de carbone, de préférence de 3 à 20 et mieux de 3 à 10 atomes de carbone, tels que l'acide maléique, l'anhydride maléique, l'acide fumarique ou l'acide itaconique.

### b) les polyesters à groupes (méth)acrylate latéraux et/ou terminaux :

Il s'agit d'un groupe de polymères de type polyester obtenus par polycondensation d'un mélange :
- de diacides carboxyliques aliphatiques, linéaires ou ramifiés, ou cycloaliphatiques comportant notamment de 3 à 50 atomes de carbone, de préférence de 3 à 20 et mieux de 3 à 10 atomes de carbone, tels que l'acide adipique ou l'acide sébacique, de diacides carboxyliques aromatiques ayant notamment de 8 à 50 atomes de carbone, de préférence de 8 à 20 et mieux de 8 à 14 atomes de carbone, tels que les acides phtaliques, notamment l'acide téréphtalique, et/ou de diacides carboxyliques issus de dimères d'acides gras à insaturation éthyléniques tels que les dimères des acides oléique ou linoléique décrits dans la demande EP 959 066 (paragraphe [0021]) commercialisés sous les dénominations Pripol^{®} par la société Unichema ou Empol^{®} par la société Henkel, tous ces diacides devant être exempts de doubles liaisons éthyléniques polymérisables,
- de diols aliphatiques linéaires ou ramifiés ou cycloaliphatiques comportant notamment de 2 à 50 atomes de carbone, de préférence de 2 à 20 et mieux de 2 à 10 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 et mieux de 6 à 15 atomes de carbone tel que le bisphénol A et le bisphénol B, et
- d'au moins un ester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.

Ces polyesters différent de ceux décrits ci-dessus sous le point a) par le fait que les doubles liaisons éthyléniques ne sont pas situées dans la chaîne principale mais sur des groupes latéraux et/ou à l'extrémité des chaînes. Ces doubles liaisons éthyléniques sont celles des groupes (méth)acrylate présents dans le polymère.

De tels polyesters sont commercialisés par exemple par la société UCB sous les dénominations EBECRYL^{®} (EBECRYL^{®} 450 : masse molaire 1600, en moyenne 6 fonctions acrylate par molécule, EBECRYL^{®} 652 : masse molaire 1500, en moyenne 6 fonctions acrylate par molécule, EBECRYL^{®} 800 : masse molaire 780, en moyenne 4 fonctions acrylate par molécule, EBECRYL^{®} 810 : masse molaire 1000, en moyenne 4 fonctions acrylate par molécule, EBECRYL^{®} 50 000 : masse molaire 1500, en moyenne 6 fonctions acrylate par molécule).

### c) les polyuréthannes et/ou polyurées à groupes (méth)acrylate, obtenus par polycondensation :

- de diisocyanates, triisocyanates et/ou polyisocyanates aliphatiques cycloaliphatiques et/ou aromatiques ayant notamment de 4 à 50 atomes de carbone, de préférence de 4 à 30 atomes de carbone, tels que l'hexaméthylènediisocyanate, l'isophoronediisocyanate, le toluènediisocyanate, le diphénylméthanediisocyanate ou les isocyanurates de formule : 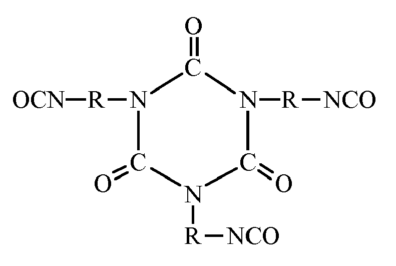 résultant de la trimérisation de 3 molécules de diisocyanates OCN-R-CNO, où R est un radical hydrocarboné linéaire, ramifié ou cyclique comportant de 2 à 30 atomes de carbone;
- de polyols, notamment de diols, exempts d'insaturations éthyléniques polymérisables, tels que le 1,4-butanediol, l'éthylèneglycol ou le triméthylolpropane, et/ou de polyamines, notamment choisi parmi les diamines aliphatiques, les diamines cycloaliphatiques, les diamines aromatiques et leurs mélanges, lesdites polyamines ayant notamment de 3 à 50 atomes de carbone, telles que l'éthylènediamine ou l'hexaméthylènediamine, et
- d'au moins un ester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.

De tels polyuréthannes/polyurées à groupes acrylates sont commercialisés par exemple sous la dénomination SR 368 (tris(2-hydroxyéthyl)isocyanurate-triacrylate) ou CRAYNOR^{®} 435 par la société CRAY VALLEY, ou sous la dénomination EBECRYL^{®} par la société UCB (EBECRYL^{®} 210 : masse molaire 1500, 2 fonctions acrylate par molécule ; EBECRYL^{®} 230 : masse molaire 5000, 2 fonctions acrylate par molécule ; EBECRYL^{®} 270 : masse molaire 1500, 2 fonctions acrylate par molécule ; EBECRYL^{®} 8402 : masse molaire 1000, 2 fonctions acrylate par molécule ; EBECRYL^{®} 8804 : masse molaire 1300, 2 fonctions acrylate par molécule ; EBECRYL^{®} 220 : masse molaire 1000, 6 fonctions acrylate par molécule ; EBECRYL^{®} 2220 : masse molaire 1200, 6 fonctions acrylate par molécule; EBECRYL^{®} 1290 : masse molaire 1000, 6 fonctions acrylate par molécule ; EBECRYL^{®} 800 : masse molaire 800, 6 fonctions acrylate par molécule).

On peut également citer les polyuréthannes aliphatiques diacrylate hydrosolubles commercialisés sous les dénominations EBECRYL^{®} 2000, EBECRYL^{®} 2001 et EBECRYL^{®} 2002, et les polyuréthannes diacrylate en dispersion aqueuse commercialisés sous les dénominations commerciales IRR^{®} 390, IRR^{®} 400, IRR^{®} 422 IRR^{®} 424 par la société UCB.

### d) les polyéthers à groupes (méth)acrylate obtenus par estérification, par l'acide (méth)acrylique, des groupes hydroxyle terminaux d'homopolymères ou de copolymères d'alkylèneglycols en C₁₋₄, tels que le polyéthylèneglycol, le polypropylèneglycol, les copolymères d'oxyde d'éthylène et d'oxyde de propylène ayant de préférence une masse moléculaire moyenne en poids inférieure à 10 000, le triméthylolpropane polyéthoxylé ou polypropoxylé.

Des polyoxyéthylènes-di(méth)acrylate de masse molaire appropriée sont commercialisés par exemple sous les dénominations SR 259, SR 344, SR 610, SR 210, SR 603 et SR 252 par la société CRAY VALLEY ou sous la dénomination EBECRYL^{®} 11 par UCB. Des triacrylates de triméthylolpropane polyéthoxylé sont commercialisés par exemple sous les dénominations SR 454, SR 498, SR 502, SR 9035, SR 415 par la société CRAY VALLEY ou sous la dénomination EBECRYL^{®} 160 par la société UCB. Des triacrylates de triméthylolpropane polypropoxylé sont commercialisés par exemple sous les dénominations SR 492 et SR 501 par la société CRAY VALLEY.

### e) les époxyacrylates obtenus par réaction entre

- au moins un diépoxyde choisi par exemple parmi :
   (i) l'éther diglycidylique de bisphénol A,
   (ii) une résine diépoxy résultant de la réaction entre l'éther diglycidylique de bisphénol A et l'épichlorhydrine,
   (iii) une résine époxyester à extrémités α,ω-diépoxy résultant de la condensation d'un diacide carboxylique ayant de 3 à 50 atomes de carbone avec un excès stoechiométrique de (i) et/ou (ii),
   (iv) une résine époxyéther à extrémités α,ω-diépoxy résultant de la condensation d'un diol ayant de 3 à 50 atomes de carbone avec un excès stoechiométrique de (i) et/ou (ii),
   (v) les huiles naturelles ou synthétiques portant au moins 2 groupes époxyde, telles que l'huile de soja époxydée, l'huile de lin époxydée et l'huile de vernonia époxydée,
   (vi) un polycondensat phénol-formaldéhyde (résine Novolac^{®}), dont les extrémités et/ou les groupes latéraux ont été époxydés, et
- un ou plusieurs acides carboxyliques ou polyacides carboxyliques comportant au moins une double liaison éthylénique en α,β du groupe carboxylique comme l'acide (méth)acrylique ou l'acide crotonique ou les esters d'acide (méth)acrylique et d'un diol ou polyol ayant de 2 à 20 atomes de carbone, de préférence de 2 à 6 atomes de carbone tels que le (méth)acrylate de 2-hydroxyéthyle.

De tels polymères sont commercialisés, par exemple, sous les dénominations SR 349, SR 601, CD 541, SR 602, SR 9036, SR 348, CD 540, SR 480, CD 9038 par la société CRAY VALLEY, sous les dénominations EBECRYL^{®} 600 et EBECRYL^{®} 609, EBECRYL^{®} 150, EBECRYL^{®} 860, EBECRYL^{®} 3702 par la société UCB et sous les dénominations PHOTOMER^{®} 3005 et PHOTOMER^{®} 3082 par la société HENKEL.

### f) les poly(méth)acrylates d'alkyle en C₁₋₅₀ (ledit alkyle étant linéaire, ramifié ou cyclique), comportant au moins deux fonctions à double liaison éthylénique portées par les chaînes hydrocarbonées latérales et/ou terminales.

De tels copolymères sont commercialisés par exemple sous les dénominations IRR^{®} 375, OTA^{®} 480 et EBECRYL^{®} 2047 par la société UCB.

### g) les polyoléfines telles que le polybutène, le polyisobutylène,

### h) les perfluoropolyéthers à groupes acrylate obtenus par estérification, par exemple par l'acide (méth)acrylique, de perfluoropolyéthers portant des groupes hydroxyle latéraux et/ou terminaux.

De tels perfluoropolyéthers α,ω-diols sont décrits notamment dans EP-A-1057849 et sont commercialisés par la société AUSIMONT sous la dénomination FOMBLIN^{®} Z DIOL.

### i) les dendrimères et polymères hyperramifiés portant des groupes terminaux (méth)acrylate ou (méth)acrylamide obtenus respectivement par estérification ou amidification de dendrimères et de polymères hyperramifiés à fonctions terminales hydroxyle ou amino, par de l'acide (méth)acrylique.

Les dendrimères sont des molécules polymères « arborescentes », c'est-à-dire très ramifiées inventées par D. A. Tomalia et son équipe au début des années 90 (Donald A. Tomalia et al., Angewandte Chemie, Int. Engl. Ed., vol. 29, n° 2, pages 138 - 175). Il s'agit de structures construites autour d'un motif central généralement polyvalent. Autour de ce motif central, sont enchaînés, selon une structure parfaitement déterminée, des motifs ramifiés d'allongement de chaîne donnant ainsi naissance à des macromolécules symétriques, monodispersées ayant une structure chimique et stéréochimique bien définie. Des dendrimères de type polyamidoamine sont commercialisés par exemple sous la dénomination STARBUST^{®} par la société DENDRITECH.

Les polymères hyper-ramifiés sont des polycondensats, généralement de type polyester, polyamide ou polyéthylénèamine, obtenus à partir de monomères multifonctionnels, qui ont une structure arborescente similaire à celle des dendrimères mais beaucoup moins régulière que celle-ci (voir par exemple WO 93/17060 et WO 96/12754).

La société PERSTORP commercialise sous la dénomination BOLTORN^{®} des polyesters hyper-ramifiés. On trouvera sous la dénomination COMBURST^{®} de la société DENDRITECH des polyéthylèneamines hyper-ramifiées. Des poly(esteramide) hyper-ramifiés à extrémités hydroxyle sont commercialisés par la société DSM sous la dénomination HYBRANE^{®}.

Ces dendrimères et polymères hyper-ramifiés estérifiés ou amidifiés par l'acide acrylique et/ou méthacrylique se distinguent des polymères décrits sous les points a) à h) ci-dessus par le très grand nombre de doubles liaisons éthyléniques présentes. Cette fonctionnalité élevée, le plus souvent supérieure à 5, les rend particulièrement utiles en leur permettant de jouer un rôle de « noeud de réticulation », c'est-à-dire de site de réticulation multiple.

On peut donc utiliser ces polymères dendritiques et hyper-ramifiés en association avec un ou plusieurs des polymères et/ou oligomères a) à h) ci-dessus.

### 1a - Composés réactifs additionnels

Lesdites première et/ou seconde compositions du kit selon l'invention peuvent comprendre, en outre, au moins un composé réactif additionnel tel que défini ci-après.

Selon un mode de réalisation, la composition (A1) du kit selon l'invention telle que définie précédemment, peut comprend en outre au moins un composé réactif additionnel et/ou la composition (A2) du kit selon l'invention telle que définie précédemment, peut comprendre en outre au moins un composé réactif additionnel.

Selon un autre mode de réalisation, ladite composition (A3) du kit selon l'invention telle que définie précédemment, peut comprendre en outre au moins un composé réactif additionnel.

Comme composé réactif additionnel, on peut citer :
- les particules organiques ou minérales comprenant à leur surface au moins 2 groupements aliphatiques insaturés, on peut citer par exemple les silices traitées en surface par exemple par des composés siliconés à groupements vinyliques tels que par exemple la silice traitée cyclotetraméthyltetravinylsiloxane,
- des composés silazanes tels que l'hexaméthyldisilazane.

### 1b - Catalyseur

La réaction d'hydrosilylation se fait en présence d'un catalyseur qui peut être présent avec au moins l'un ou l'autre des composés (X) ou (Y) dans une même composition ou être présent de manière isolée dans une composition exempte ou non des composés (X) et (Y).

Ainsi lesdites première et/ou seconde compositions du kit selon l'invention, par exemple, comprenant respectivement les composés (X) et (Y), peuvent comprendre, en outre, au moins un catalyseur tel que défini ci-après.

Selon un mode de réalisation, la composition (A1) du kit selon l'invention telle que définie précédemment, peut comprendre outre au moins un composés (X), au moins un catalyseur et éventuellement un composé réactif additionnel et/ou la composition (A2) du kit selon l'invention telle que définie précédemment, peut comprendre outre au moins un composés (Y), au moins un catalyseur et éventuellement un composé réactif additionnel.

Selon un autre mode de réalisation, la composition (A3) du kit selon l'invention telle que définie précédemment, peut comprendre outre les composés (X) et (Y), au moins un catalyseur et éventuellement au moins un composé réactif additionnel, mais l'un au moins des composés (X), (Y) et du catalyseur étant sous forme encapsulée.

Selon un mode de réalisation particulier, le catalyseur peut être présent dans la composition (A3) sous une forme encapsulée si les deux composés (X) et (Y), dont il doit provoquer l'interaction, sont présents dans cette même composition sous une forme non encapsulée ou à l'inverse il peut y être présent dans la composition (A3) sous une forme non encapsulée si au moins l'un des composés (X) et (Y) est présent dans cette même composition sous une forme encapsulée.

Selon un autre mode de réalisation, le catalyseur est compris dans une composition (A4) du kit selon l'invention, ladite composition étant exempte des composés (X) et (Y).

Le catalyseur est de préférence à base de platine ou d'étain. On peut citer par exemple les catalyseurs à base de platine déposé sur un support de gel de silice ou de poudre de charcoal (charbon), le chlorure de platine, les sels de platine et d'acides chloroplatiniques.

On utilise, de préférence, les acides chloroplatiniques sous forme hexahydrate ou anhydre, facilement dispersible dans les milieux organosiliconés.

On peut également citer les complexes de platine tels que ceux à base d'acide chloroplatinique hexahydrate et de divinyl tetraméthyldisiloxane.

Le catalyseur peut être présent en une teneur allant de 0,0001 % à 20 % en poids par rapport au poids total de la composition le comprenant.

Les composés (X) et/ou (Y) peuvent être associés à des inhibiteurs ou retardateurs de polymérisation, et plus particulièrement des inhibiteurs du catalyseur. De façon non limitative, on peut citer les polyméthylvinylsiloxanes cycliques, et en particulier le tetravinyl tétraméthyl cyclotetrasiloxane, les alcools acétyléniques, de préférence volatils, tels que le méthylisobutynol.

La présence de sels ioniques, tels que l'acétate de sodium peut avoir une influence dans la vitesse de polymérisation des composés (X) et (Y).

A titre d'exemple d'une combinaison de composés (X) et (Y) réagissant par hydrosilylation en présence d'un catalyseur, on peut citer les références suivantes proposée par la société Dow Corning : DC 7-9800 Soft Skin Adhesive Parts A & B.

De façon avantageuse, les composés (X) et (Y) sont choisis parmi les composés siliconés susceptibles de réagir par hydrosilylation en présence d'un catalyseur ; en particulier le composé (X) est choisi parmi les polyorganosiloxanes comprenant des unités de formule (I) décrits ci-dessus et le composé (Y) est choisi parmi les organosiloxanes comprenant des unités alkylhydrogénosiloxanes de formule (III) décrits ci-dessus.

Selon un mode de réalisation particulier, le composé (X) est un polydiméthylsiloxane à groupements vinyliques terminaux, et le composé (Y) est un polyméthylhydrogénosiloxane.

### 2 - Composés (X) et (Y) susceptibles de réagir par condensation

La réaction de condensation a lieu entre au moins un composé (X), au moins un composé (Y) et éventuellement au moins un catalyseur.

Les composés (X) et (Y) peuvent être présents respectivement au sein du kit selon l'invention dans une première composition et dans une seconde composition, voire dans une autre composition que lesdites première et seconde compositions.

Selon un mode de réalisation, le composé (X) avec éventuellement au moins un catalyseur est présent dans une composition (A1) du kit selon l'invention et le composé (Y) avec éventuellement au moins un catalyseur est présent dans une composition (A2) du kit selon l'invention.

Selon un autre mode de réalisation, les composés (X) et (Y) distincts l'un de l'autre et éventuellement au moins un catalyseur sont présents dans une composition (A3) du kit selon l'invention à la condition que l'un au moins des composés (X) et (Y) soit sous forme encapsulée.

Les composés (X) et (Y) sont susceptibles de réagir par condensation, soit en présence d'eau (hydrolyse) par réaction de 2 composés porteurs de groupements alcoxysilanes, soit par condensation dite « directe » par réaction d'un composé porteur de groupement(s) alcoxysilane(s) et d'un composé porteur de groupement(s) silanol(s) ou par réaction de 2 composés porteurs de groupement(s) silanol(s).

Lorsque la condensation se fait en présence d'eau, celle-ci peut être en particulier l'humidité ambiante, l'eau résiduelle des cils, ou l'eau apportée par une source extérieure, par exemple par humidification préalable de la matière kératinique (par exemple par un brumisateur, des larmes naturelles ou artificielles).

Dans ce mode de réaction par condensation, les composés (X) et (Y), identiques ou différents, peuvent donc être choisis parmi les composés siliconés dont la chaîne principale comprend au moins deux groupes alcoxysilane et/ou au moins deux groupes silanol (Si-OH), latéraux et/ou en bout de chaîne.

Selon un mode de réalisation, le composé (X) et/ou le composé (Y) est porteur d'au moins un groupe polaire, tel que décrit ci-dessus, susceptible de former au moins une liaison hydrogène avec les matières kératiniques et plus particulièrement la peau.

Selon un mode de réalisation avantageux, les composés (X) et/ou (Y) sont choisis parmi les polyorganosiloxanes comprenant au moins deux groupes alcoxysilane. Par groupe « alcoxysilane », on entend un groupe comprenant au moins une partie -Si-OR, R étant un groupe alkyle comprenant de 1 à 6 atomes de carbone.

Les composés (X) et (Y) sont notamment choisis parmi les polyorganosiloxanes comprenant des groupes terminaux alcoxysilanes, plus spécifiquement ceux qui comprennent au moins 2 groupes alcoxysilanes terminaux, de préférence trialcoxysilanes terminaux.

Ces composés (X) et/ou (Y), identiques ou différents, comprennent de préférence de façon majoritaire des unités de formule (IV):

R⁹ₛSiO_{(4-s)/2:} (IV)

dans laquelle les groupes R⁹ représentent indépendamment les uns des autres un radical choisi parmi les groupes alkyle comprenant de 1 à 6 atomes de carbone, le phényle, les groupes fluoroalkyle, et s est égal à 0, 1, 2 ou 3. De préférence, les groupes R⁹ représentent indépendamment les uns des autres un groupe alkyle comprenant de 1 à 6 atomes de carbone. Comme groupe alkyle, on peut citer notamment le méthyle, le propyle, le butyle, l'hexyle et leurs mélanges, de préférence le méthyle ou l'éthyle. Comme groupe fluoroalkyle, on peut citer le 3, 3, 3-trifluoropropyle.

Selon un mode de réalisation particulier, les composés (X) et (Y), identiques ou différents, sont des polyorganosiloxanes comprenant des unités de formule (V) :

(R⁹₂SiO₂)₁ - (V)

dans laquelle R⁹ est tel que décrit ci-dessus, de préférence R⁹ est un radical méthyle, et f est tel que le polymère présente avantageusement une viscosité à 25 °C allant de 0,5 à 3000 Pa.s, de préférence allant de 5 à 150 Pa.s ; par exemple f peut aller de 2 à 5000, de préférence de 3 à 3000, et préférentiellement de 5 à 1000, bornes incluses.

Ces composés (X) et/ou (Y) polyorganosiloxanes comprennent au moins 2 groupes trialcoxysilanes terminaux par molécule de polymère, lesdits groupes ayant la formule (VI) suivante :

- ZSIR¹ₓ(OR)₃₋ₓ⁻ (VI)

dans laquelle :
- les radicaux R sont choisis indépendamment l'un de l'autre, parmi un groupe méthyle, éthyle, n-propyle, isopropyle, n-butyle, sec-butyle, isobutyle, de préférence un groupe méthyle ou éthyle,
- R¹ est un groupe méthyle ou éthyle,
- x est égal à 0 ou 1, de préférence x est égal à 0 et
- Z est choisi parmi les groupes hydrocarbonés divalents ne comportant pas d'insaturation éthylénique et comprenant de 1 à 18 atomes de carbone, de préférence de 2 à 18 atomes de carbone (groupes alkylène), les combinaisons de radicaux hydrocarbonés divalents et de segments siloxanes de formule (IX) suivante : 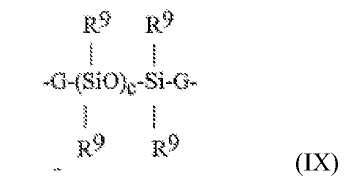
R⁹ étant tel que décrit plus haut, G est un radical hydrocarboné divalent ne comportant pas d'insaturation éthylénique et comprenant de 1 à 18 atomes de carbone, de préférence de 2 à 18 atomes de carbone et c est un entier allant de 1 à 6.

Z et G peuvent être notamment choisis parmi les groupements alkylènes tels que le méthylène, l'éthylène, le propylène, le butylène, le pentylène, l'hexylène, les groupements arylène tels que le phénylène.

De préférence, Z est un groupe alkylène, et mieux éthylène.

Ces polymères peuvent présenter en moyenne au moins 1,2 groupements terminaux ou chaînes terminales trialcoxysilanes par molécule, et de préférence en moyenne au moins 1,5 groupements terminaux trialcoxysilanes par molécule. Ces polymères pouvant présenter au moins 1,2 groupements terminaux trialcoxysilanes par molécule, certains peuvent comprendre d'autre types de groupes terminaux tels que des groupements terminaux de formule CH₂=CH-SiR⁹₂- ou de formule R⁶₃-Si-, dans laquelle R⁹ est tel que défini plus haut et chaque groupe R⁶ est indépendamment choisi parmi les groupes R⁹ ou vinyle. On peut citer comme exemples de tels groupements terminaux les groupes triméthoxysilane, triéthoxysilane, vinyldiméthoxysilane et vinylméthyloxyphénylsilane.

De tels polymères sont notamment décrits dans les documents US 3 175 993, US 4 772 675, US 4 871 827, US 4 888 380, US 4 898 910, US 4 906 719 et US 4 962 174 dont le contenu est incorporé par référence au présent texte.

On peut citer à titre de composé (X) et/ou (Y) en particulier les polyorganosiloxanes choisis parmi les polymères de formule (VII) : 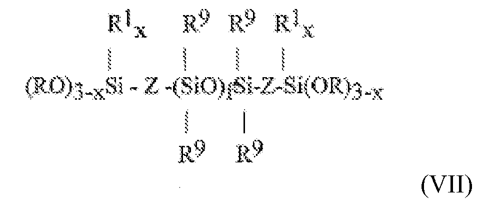 dans laquelle R, R¹, R⁹, Z, x et f sont tels que décrits plus haut.

Les composés (X) et/ou (Y) peuvent également comprendre un mélange de polymères de formule (VII) ci-dessus avec des polymères de formule (VIII) suivante : 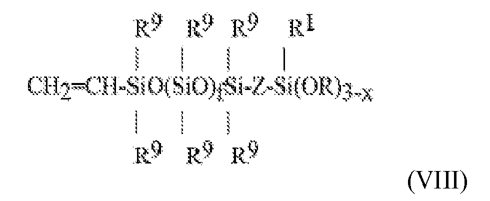 dans laquelle R, R¹, R⁹, Z, x et f sont tels que décrits plus haut.

Lorsque le composé (X) et/ou (Y) polyorganosiloxanes à groupe(s) alcoxysilane(s) comprend un tel mélange, les différents polyorganosiloxanes sont présents en des teneurs telles que les chaînes organosilyles terminales représentent moins de 40 %, de préférence moins de 25 % en nombre des chaînes terminales.

Les composés (X) et/ou (Y) polyorganosiloxanes particulièrement préférés sont ceux de formule (VII) décrits ci-dessus. De tels composés (X) et/ou (Y) sont décrits par exemple dans le document WO 01/96450.

Comme indiqué plus haut, les composés (X) et (Y) peuvent être identiques ou différents.

En particulier, les composés (X) et (Y) peuvent représenter un mélange de polydiméthylsiloxanes à groupements méthoxysilanes.

Selon une variante, l'un des 2 composés réactifs (X) ou (Y), est de nature siliconée et l'autre est de nature organique. Par exemple le composé (X) est choisi parmi les oligomères ou polymères organiques ou les oligomères ou polymères hybrides organique/silicone, lesdits polymères ou oligomères comprenant au moins deux groupements alcoxysilanes et (Y) est choisi parmi les composés siliconés tels que les polyorganosiloxanes décrits ci-dessus. En particulier, les oligomères ou polymères organiques sont choisis parmi les oligomères ou polymères vinyliques, (méth)acryliques, polyesters, polyamides, polyuréthanes et/ou polyurées, polyéthers, polyoléfines, perfluoropolyéthers, dendrimères et polymères hyper-ramifiés organiques, et leurs mélanges.

Selon un mode de réalisation, le composé (X) de nature organique ou de nature hybride organique/silicone est porteur d'au moins un groupe polaire, tel que décrit ci-dessus, susceptible de former au moins une liaison hydrogène avec les matières kératiniques, et plus particulièrement la peau.

Les polymères organiques de nature vinylique ou (méth)acryliques, porteurs de groupes latéraux alcoxysilanes, pourront en particulier être obtenus par copolymérisation d'au moins un monomère organique vinylique ou (méth)acrylique avec un (méth)acryloxypropyltriméthoxysilane, un vinyl triméthoxysilane, un vinyltriéthoxysilane, un allyltriméthoxysilanes etc..

On peut citer par exemple, les polymères (méth)acryliques décrits dans le document de KUSABE.M, Pitture e Verniei - European Coating ; 12-B, pages 43-49, 2005, et notamment les polyacrylates à groupes alcoxysilanes référencés MAX de Kaneka ou ceux décrits dans la publication de PROBSTER, M, Adhesion-Kleben & Dichten, 2004, 481 (1-2), pages 12-14.

Les polymères organiques résultant d'une polycondensation ou d'une polyaddition, tels que les polyesters, polyamides, polyuréthanes et/ou polyurées, polyéthers, et porteurs de groupes alcoysilanes latéraux et/ou terminaux, pourront résulter par exemple de la réaction d'un prépolymère oligomère tel que décrit plus haut avec l'un des co-réactifs silanes suivant porteurs d'au moins un groupe alcoxysilane : aminopropyltriméthoxysilane, aminopropyltriéthoxysilane, aminoéthyl aminopropyl triméthoxysilane, glycidoxypropyltriméthoxysilane, glycidoxypropyltriéthoxysilane, époxycyclohéxyléthyltriméthoxysilane, mercaptopropyltriméthoxysilane.

Des exemples de polyéthers et de polyisobutylènes à groupes alcoxysilanes sont décrits dans la publication de KUSABE.M., Pitture e Verniei - European Coating ; 12-B, pages 43-49, 2005. Comme exemple de polyuréthanes à groupes alcoxysilanes terminaux, on peut citer ceux décrits dans le document PROBSTER, M., Adhesion-Kleben & Dichten, 2004, 481 (1-2), pages 12-14 ou encore ceux décrits dans le document LANDON, S., Pitture e Verniei vol. 73, N° 11, pages 18-24, 1997 ou dans le document HUANG, Mowo, Pitture e Verniei vol. 5, 2000, pages 61-67, on peut notamment citer les polyuréthanes à groupes alcoxysilanes de OSI-WITCO-GE.

A titre de composés (X) et/ou (Y) polyorganosiloxane, on peut citer les résines de type MQ ou MT portant elles-même des extrémités alcoxysilanes et/ou silanols comme par exemple les résines poly(isobutylsilsesquioxane) fonctionnalisées par des groupes silanols proposées sous la référence SST-S7C41 (3 groupes Si-OH) par la société Gelest.

### 2a - Composé réactif additionnel

Lesdites première et/ou seconde compositions du kit selon l'invention peuvent comprendre, en outre, au moins un composé réactif additionnel tel que défini ci-après.

Selon un mode de réalisation, la composition (A1) du kit selon l'invention, telle que définie précédemment, peut comprendre en outre au moins un composé réactif additionnel et/ou la composition (A2) du kit selon l'invention, telle que définie précédemment, peut comprendre, en outre, au moins un composé réactif additionnel.

Selon un autre mode de réalisation, ladite composition (A3) du kit selon l'invention, telle que définie précédemment, peut comprendre, en outre, au moins un composé réactif additionnel.

Selon un mode de réalisation, le composé (X) et/ou (Y) peut être associé en outre à un composé réactif additionnel comprenant au moins deux groupes alcoxysilane et/ou silanol.

Comme composé réactif additionnel, on peut citer, par exemple une ou des particules organiques ou minérales comprenant à leur surface des groupements alcoxysilanes et/ou silanols, par exemple des charges traitées en surface par de tels groupes.

### 2b - Catalyseur

La réaction de condensation peut se faire en présence d'un catalyseur à base de métal qui peut être présent avec au moins l'un ou l'autre des composés (X) ou (Y) ou être présent de manière isolée dans une composition exempte ou non des composés (X) et (Y).

Ainsi lesdites première et/ou seconde compositions du kit selon l'invention, par exemple, comprenant respectivement les composés (X) et (Y), peuvent comprendre, en outre, au moins un catalyseur tel que défini ci-après.

Selon un mode de réalisation, la composition (A1) du kit selon l'invention telle que définie précédemment peut comprendre outre au moins un composés (X), au moins un catalyseur et éventuellement un composé réactif additionnel et/ou la composition (A2) du kit selon l'invention telle que définie précédemment, peut comprendre outre au moins un composés (Y), au moins un catalyseur et éventuellement un composé réactif additionnel.

Selon un autre mode de réalisation, la composition (A3) du kit selon l'invention telle que définie précédemment peut comprendre outre les composés (X) et (Y), au moins un catalyseur et éventuellement au moins un composé réactif additionnel, mais l'un au moins des composés (X), (Y) et éventuellement du catalyseur étant sous forme encapsulée.

Selon un mode de réalisation particulier, le catalyseur peut être présent dans la composition (A3) sous une forme encapsulée si les deux composés (X) et (Y), dont il doit provoquer l'interaction, sont présents dans cette même composition sous une forme non encapsulée ou à l'inverse il peut y être présent dans la composition (A3) sous une forme non encapsulée si au moins l'un des composés (X) et (Y) est présent dans cette même composition sous une forme encapsulée.

Selon un autre mode de réalisation, le catalyseur est compris dans une composition (A4) du kit selon l'invention, ladite composition étant exempte des composés (X) et (Y).

Le catalyseur qui peut être utile dans ce type de réaction est de préférence un catalyseur à base de titane. On peut citer notamment les catalyseurs à base de tetraalcoxytitane de formule (XI) :

Ti(OR²)_{y}(OR³)_{4-y}, (XI)

dans laquelle R² est choisi parmi les radicaux alkyle tertiaires tels que le tert butyle, le tert amyle et le 2,4-diméthyl-3-pentyle ; R³ représente un radical alkyle comprenant de 1 à 6 atomes de carbone, de préférence un groupe méthyle, éthyle, n-propyle, isopropyle, n-butyle, sec-butyle, hexyle et y est un nombre allant de 3 à 4, mieux de 3,4 à 4.

Le catalyseur peut être présent en une teneur allant de 0,0001 % à 20 % en poids par rapport au poids total de la composition le contenant.

### 2c - Diluant

Les compositions utiles comprenant (X) et/ou (Y) peuvent comprendre en outre une huile siliconée volatile (ou diluant) destinée à faire diminuer la viscosité de la composition. Lesdites première et/ou seconde compositions du kit selon l'invention peuvent comprendre, en outre, au moins une huile siliconée volatile telle que définie ci-après.

Selon un mode de réalisation, la composition (A1) du kit selon l'invention telle que définie précédemment peut comprendre en outre au moins une huile siliconée volatile et/ou la composition (A2) du kit selon l'invention telle que définie précédemment, peut comprendre en outre au moins une huile siliconée volatile.

Selon un autre mode de réalisation, la composition (A3) du kit selon l'invention telle que définie précédemment, peut comprendre en outre au moins une huile siliconée volatile.

Cette huile peut être choisie parmi les silicones linéaires à chaîne courte telles que l'hexaméthyldisiloxane, l'octaméthyltrisiloxane, les silicones cycliques telles que l'octaméthylcyclotetrasiloxane, la decaméthylclopentasiloxane et leurs mélanges.

Cette huile siliconée peut représenter de 5 % à 95 %, de préférence de 10 à 80 % en poids par rapport au poids total de l'ensemble des compositions dans ledit kit selon l'invention.

A titre d'exemple de combinaison d'une composition (A1) ou (A2) contenant des composés (X) et (Y) porteurs de groupements alcoxysilanes et réagissant par condensation et d'une composition (A4) exempte de composés (X) et (Y), on peut citer la combinaison suivante détaillée dans le tableau 1 ci-dessous et préparée par la société Dow Corning. Dans cette combinaison, on distingue les composés (X) et (Y), identiques, et donc présents dans une même composition (la composition (A1) = composition (A2)) et le catalyseur présent dans une composition (A4).

**Tableau 1**

| Ingrédient (Nom INCI) | N°CAS | Teneurs (%) | Fonction |
|---|---|---|---|
| Composition (A1)= Composition (A2) | | | |
| Bis-Triméthoxysiloxyéthyl Tétraméthyldisiloxyéthyl Diméthicone | PMN87176 | 25-45 | Polymère |
| Silica Silylate | 68909-20-6 | 5-20 | Charge |
| Disiloxane | 107-46-0 | 30-70 | Solvant |

| Composition (A4) | | | |
|---|---|---|---|
| Disiloxane | 107-46-0 | 80-99 | Solvant |
| Tétra T Butyl Titanate | | 1-20 | Catalyseur |

### 3/ Réticulation en présence de peroxyde:

La réaction de réticulation a lieu entre au moins un composé (X), au moins un composé (Y), en présence d'au moins un peroxyde et en présence, éventuellement, d'au moins un catalyseur.

Les composés (X) et (Y) peuvent être présents respectivement au sein du kit selon l'invention dans une première composition et dans une seconde composition, voire dans une autre composition que lesdites première et seconde compositions.

Selon un mode de réalisation particulier, le composé (X) est présent dans une composition (A1) et le composé (Y) est présent dans une composition (A2).

Selon un autre mode de réalisation, les composés (X) et (Y) distincts l'un de l'autre sont présents dans une composition (A3).

La réaction de réticulation se fait en présence d'un peroxyde qui peut être présent avec au moins l'un ou l'autre des composés (X) ou (Y) dans une même composition ou être présent de manière isolée dans une composition exempte ou non des composés (X) et (Y).

Ainsi lesdites première et/ou seconde compositions du kit selon l'invention comprenant respectivement les composés (X) et (Y) peuvent comprendre, en outre, au moins un peroxyde tel que défini ci-après.

Selon un mode de réalisation, la composition (A1) du kit selon l'invention telle que définie précédemment, peut comprendre outre au moins un composés (X), au moins un peroxyde et éventuellement un au moins un catalyseur et/ou la composition (A2) du kit selon l'invention telle que définie précédemment, peut comprendre outre au moins un composés (Y), au moins un peroxyde et éventuellement au moins un catalyseur.

Selon un autre mode de réalisation, la composition (A3) du kit selon l'invention telle que définie précédemment, peut comprendre outre les composés (X) et (Y), au moins un peroxyde et éventuellement au moins catalyseur, mais l'un au moins des composés (X), (Y) et du peroxyde étant sous forme encapsulée, sachant que les composés (X), (Y) et le peroxyde ne sont jamais présents simultanément dans la même composition autre que celle formant le film selon l'invention.

Selon un mode de réalisation particulier, le peroxyde peut être présent dans la composition (A3) sous une forme encapsulée si les deux composés (X) et (Y), dont il doit provoquer l'interaction, sont présents dans cette même composition sous une forme non encapsulée ou à l'inverse il peut y être présent dans la composition (A3) sous une forme non encapsulée si au moins l'un des composés (X) et (Y) est présent dans cette même composition sous une forme encapsulée.

Selon un autre mode de réalisation, le peroxyde est compris dans une composition (A4) du kit selon l'invention, ladite composition étant exempte des composés (X) et (Y).

Cette réaction se fait de préférence par chauffage à une température supérieure ou égale à 50 °C, de préférence supérieure ou égale à 80 °C, allant jusqu'à 120 °C.

Les composés (X) et (Y), identiques ou différents, comprennent dans ce cas au moins deux groupements latéraux -CH₃ et/ou au moins deux chaînes latérales portant un groupement -CH₃.

Les composés (X) et (Y) sont de préférence siliconés et peuvent être choisis par exemple parmi les polydiméthylsiloxanes linéaires non volatiles de haut poids moléculaire, ayant un degré de polymérisation supérieur à 6 présentant au moins deux groupements latéraux -CH₃ reliés à l'atome de silicium et/ou au moins deux chaînes latérales portant un groupement -CH₃. On peut citer par exemple les polymères décrits dans le Catalogue « Reactive Silicones » de la société Gelest Inc., Edition 2004, page 6, et notamment les copolymères (aussi appelés gommes) de vinylméthylsiloxane-diméthylsiloxane de poids moléculaires allant de 500 000 à 900 000 et notamment de viscosité supérieure à 2 000 000 cSt.

A titre de peroxydes utilisables dans le cadre de l'invention, on peut citer le peroxyde de benzoyle, le peroxyde de 2,4-dichlorobenzoyle et leurs mélanges.

Selon un mode de réalisation, la réaction d'hydrosilylation en présence d'un catalyseur, ou la réaction de condensation, ou bien encore la réaction de réticulation en présence d'un peroxyde, entre les composés (X) et (Y) est accélérée par un apport de chaleur en élevant par exemple la température du système entre 25 °C et 180 °C.

D'une façon générale, quel que soit le type de réaction par laquelle les composés (X) et (Y) réagissent ensemble, le pourcentage molaire de (X) par rapport à l'ensemble des composés (X) et (Y), c'est-à-dire le ratio (X)/[(X)+(Y)] x 100, peut varier de 5 % à 95 %, de préférence de 10 % à 90 %, mieux encore de 20 % à 80 %.

De même, le pourcentage molaire de (Y) par rapport à l'ensemble des composés (X) et (Y), c'est-à-dire le ratio (Y)/[(X)+(Y)] x 100, peut varier de 5 % à 95 %, de préférence de 10 % à 90 %, mieux encore de 20 % à 80 %.

Le composé (X) peut présenter une masse moléculaire moyenne en poids (Mw) allant de 150 à 1 000 000, de préférence de 200 à 800 000, de préférence encore de 200 à 250 000.

Le composé (Y) peut présenter une masse moléculaire moyenne en poids (Mw) allant de 200 à 1 000 000, de préférence de 300 à 800 000, de préférence encore de 500 à 250 000.

Le composé (X) peut représenter de 0,1 % à 95 % en poids par rapport au poids total de la composition le contenant, de préférence de 1 % à 90 %, et mieux de 5%à80%.

Le composé (Y) peut représenter de 0,1 % à 95 % en poids par rapport au poids total de la composition le contenant, de préférence de 1 % à 90 % et mieux de 5 % à 80 %.

Le ratio entre les composés (X) et (Y) peut varier de manière à moduler la vitesse de réaction et donc la vitesse de formation du film ou encore de manière à adapter les propriétés du film formé (par exemple ses propriétés adhésives) selon l'application recherchée.

En particulier, les composés (X) et (Y) peuvent être présents en un ratio (X)/(Y) molaire allant de 0,05 à 20 et mieux de 0,1 à 10.

Comme précisé précédemment, selon un mode de réalisation de l'invention, les composés (X) et (Y) peuvent être mis en oeuvre sous la forme d'une unique composition (A3) qui peut contenir le composé (X) et/ou le composé (Y) et/ou le cas échéant, suivant le type de réaction envisagée entre les composés (X) et (Y), le catalyseur et/ou le peroxyde sous forme encapsulée.

Lorsque le catalyseur et/ou le peroxyde sont sous forme encapsulés, ils peuvent être alors :
(i) soit encapsulés indépendamment l'un de l'autre avec (X) ou avec (Y),
(ii) soit encapsulés seuls, indépendamment l'un de l'autre et indépendamment des autres composés de la réaction tels que les composés (X) et (Y),
(iii) soit encapsulés ensemble indépendamment des autres composés de la réaction tels que les composés (X) et (Y) ou alors soit encapsulés ensemble et avec l'un des deux composés (X) ou (Y).

Selon un mode de réalisation particulier, une seule composition (A3), contenant dans un milieu physiologiquement acceptable le ou les composés (X) et le ou les composés (Y) est appliquée sur les matières kératiniques, en particulier la peau, avec au moins un des composés (X) et (Y) sous une forme encapsulée.

Selon une autre variante de réalisation, les composés (X) et (Y) sont encapsulés séparément. Ainsi les composés (X) et (Y) peuvent être conditionnés dans une même composition (A3) tout en s'affranchissant du risque de réaction prématuré entre eux. La réaction n'intervient alors qu'au moment où les capsules sont rompues, c'est-à-dire extemporanément à l'application ou au moment de l'application sur les matières kératiniques, en particulier la peau.

Dans le cadre de la présente invention, sont plus particulièrement considérées les formes encapsulées de type coeur/écorce dites encore microcapsules dont l'écorce est de nature polymérique et le coeur contient le composé (X) ou le composé (Y), l'un de ses composés (X) et (Y) étant le cas échéant encapsulé avec le catalyseur ou le peroxyde si nécessaire à l'interaction des deux composés. Dans l'hypothèse où ce catalyseur n'est pas encapsulé avec l'un ou l'autre des composés (X) ou (Y), il est présent dans la composition cosmétique contenant les formes encapsulées.

De nombreuses techniques sont, à ce jour, disponibles pour préparer ce type de microcapsules comme celles obtenues par une technique dite basculement de solvant notamment illustrée dans les documents EP 274 961 et EP 1 552 820.

Plus particulièrement, l'écorce des capsules de composé (X) ou (Y), mises en oeuvre selon l'invention, est de nature polymérique, non réticulée, non hydrosoluble et non soluble dans le coeur des capsules.

D'une manière générale, tous les polymères, d'origine naturelle ou synthétique, solubles dans un solvant non miscible à l'eau et notamment ceux ayant un point de fusion inférieur au point d'ébullition de l'eau à la pression atmosphérique (100 °C), peuvent convenir.

Ces polymères peuvent être biodégradables, comme par exemple les polyesters, ou non.

A titre illustratif des polymères convenant à l'invention, on peut notamment citer :
- les polymères de cyanoacrylate d'alkyle en C₂-C₁₂
- les polymères formés par les poly-L-lactides, les poly-DL-lactides, les polyglycolides et les copolymères correspondants,
- les polycaprolactones,
- les polymères de l'acide 3-hydroxy butyrique,
- les copolymères de chlorure de vinyle et d'acétate de vinyle,
- les copolymères d'acide et d'ester méthacrylique, notamment d'acide méthacrylique et d'ester d'acide méthacrylique,
- l'acéto-phtalate de polyvinyle,
- l'acéto-phtalate de cellulose,
- le copolymère polyvinylpyrrolidone-acétate de vinyle,
- les polyéthylènevinylacétates,
- les polyacrylonitriles,
- les polyacrylamides,
- les polyéthylèneglycols,
- les poly-(méthacrylate d'hydroxyalkyle en C₁ à C₄),
- les esters de cellulose et d'acide carboxylique en C₁-C₄,
- le polystyrène et les copolymères de styrène et d'anhydride maléique, les copolymères de styrène et d'acide acrylique, les terpolymères séquencés styrène éthylène/butylène-styrène, les terpolymères séquencés styrène-éthylène/propylène-styrène,
- les oligomères styrène alkylalcool,
- les terpolymères d'éthylène, d'acétate de vinyle et d'anhydride maléique,
- les polyamides,
- les polyéthylènes,
- les polypropylènes,
- les organopolysiloxanes dont les polydiméthylsiloxanes,
- les poly (alkylène adipate),
- les polyesters polyol,
- les polymères siliconés de polysilsesquioxane, ,
- les polyesters dendritiques à fonction hydroxyle terminale,
- les polymères hydrodipersibles mais néanmoins solubles dans des solvants non miscibles à l'eau comme par exemple : les polyesters, poly(ester amides), polyuréthannes et copolymères vinyliques portant des fonctions acide carboxylique et/ou sulfonique et en particulier ceux décrits dans le document FR 2 787 729,
- les copolymères blocs insolubles dans l'eau à température ambiante et solides à température ambiante, ayant au moins un bloc d'un des polymères précédents, et
- leurs mélanges.

Ces polymères ou copolymères peuvent posséder un poids moléculaire moyen compris entre 1000 et 500 000 et en particulier entre 1500 et 100 000.

Conviennent tout particulièrement à l'invention, les poly(alkylène adipate), les organo polysiloxanes, les polycaprolactones, l'acétophtalate de cellulose, l'acétobutyrate de cellulose, les esters de cellulose, le polystyrène, et ses dérivés, et notamment les polycaprolactones.

Bien entendu, l'homme du métier est à même, de par ses connaissances, d'ajuster le poids moléculaire du polymère sélectionné vis-à-vis de sa concentration dans le solvant afin d'avoir une viscosité du mélange compatible avec une émulsification satisfaisante.

En ce qui concerne le coeur lipophile, il peut contenir outre le composé (X) ou le composé (Y), au moins une huile. L'huile peut être choisie parmi les huiles décrites ci-après pour la phase huileuse. L'huile est de préférence une huile siliconée.

En ce qui concerne le protocole opératoire pour préparer des capsules convenant à l'invention, l'homme de l'art pourra se reporter notamment à l'enseignement du document EP 1 552 820 cité précédemment. Le choix des tensioactifs requis ainsi que la mise en oeuvre du procédé fait appel aux connaissances de l'homme de l'art.

Selon un mode de réalisation de l'invention, les première et/ou seconde compositions, en particulier les compositions (A1) et (A2), voir la composition (A3) peuvent comprendre un agent plastifiant favorisant la formation d'un film avec le produit de réaction entre les composés (X) et (Y). Un tel agent plastifiant peut être choisi parmi tous les composés connus de l'homme du métier comme étant susceptibles de remplir la fonction recherchée.

Outre les composés (X) et (Y), le kit comprend au moins un filtre solaire organique hydrophobe tel que défini ci-après.

### FILTRES SOLAIRES OU PHOTOPROTECTEURS ORGANIQUES HYDROPHOBES

Le kit selon l'invention comprend ainsi en outre au moins un système filtrant hydrophobe comprenant :
(i) au moins un filtre solaire organique hydrophobe (A) capable d'absorber les rayonnements UV de 320 à 400 nm et au moins un filtre solaire organique hydrophobe (B) capable d'absorber les rayonnements UV de 280 à 320 nm et/ou au moins un filtre minéral (D) ;
(ii) au moins un filtre solaire organique hydrophobe (C) capable d'absorber simultanément les rayonnements UV de 280 à 320 nm et 320 à 400 nm, et éventuellement un filtre minéral (D) ; ou
(iii) au moins un filtre solaire organique hydrophobe (A) capable d'absorber les rayonnements UV de 320 à 400 nm et au moins soit un filtre solaire organique hydrophobe (C) capable d'absorber simultanément les rayonnements UV de 280 à 320 nm et 320 à 400 nm, soit un filtre minéral (D) ;

Le terme « filtre solaire» est équivalent, dans le cadre de l'invention, au terme « filtre photoprotecteur ».

Lesdites première et/ou seconde compositions du kit selon l'invention ainsi que les compositions (A1), (A2), (A3) et (A4) peuvent en outre comprendre au moins un système filtrant hydrophobe tel que défini ci-dessus et plus en détail ci-après.

Selon un mode de réalisation du kit selon l'invention, la composition (A1) telle que définie ci-dessus peut comprendre en outre au moins le système filtrant hydrophobe tel que décrit précédemment et/ou la composition (A2) telle que définie ci-dessus peut comprendre en outre le système filtrant hydrophobe tel que décrit précédemment.

Selon un autre mode de réalisation du kit selon l'invention, ladite composition (A3) telle que définie ci-dessus comprend en outre au moins le système filtrant hydrophobe tel que décrit précédemment.

Selon un mode de réalisation préféré, la composition (A2) comprend le composant (Y) et le système filtrant hydrophobe conforme à l'invention.

Dans le cadre de la présente invention, on entend par « filtre solaire organique hydrophobe » un composé organique filtrant des radiations UV ayant une solubilité à 25 °C dans l'eau inférieure ou égale à 0,5 % en poids, cette solubilité étant définie comme la quantité de produit en solution dans le solvant à l'équilibre avec un excès de solide en suspension. Le terme « filtre solaire insoluble dans l'eau » est équivalent, dans le cadre de l'invention, au terme « filtre solaire hydrophobe»

A contrario, un « filtre solaire organique hydrophile » est un composé organique ne répondant pas à cette définition. Le terme « filtre solaire soluble dans l'eau» est équivalent, dans le cadre de l'invention au terme « filtre solaire soluble dans l'eau ».

Les filtres organiques hydrophobes (A), (B) et (C) peuvent notamment être choisis parmi différentes familles de composés chimiques. On peut notamment citer les dérivés de l'acide paraaminobenzoique, les dérivés salicyliques, les dérivés cinnamiques, les benzophénones et aminobenzophénones, les dérivés anthraniliques, les dérivés du dibenzoylméthane, les dérivés de β,β'-diphénylacrylate, les dérivés du benzylidène camphre, les dérivés du phényl benzotriazole, les dérivés triazine, les bis-résorcinyl triazines, les dérivés d'imidazolines, les dérivés du benzalmalonate, les dérivés de 4,4-diarylbutadiène, les dérivés de benzoxazole, les mérocyanines et leurs mélanges.

### Filtres hydrophobes (A) capables d'absorber les UV de 320 à 400 nm

### Dérivés du dibenzoylméthane :

- Butyl Methoxydibenzoylméthane vendu notamment sous le nom commercial « PARSOL 1789 » par DSM Nutritional Products, Inc ;
- Isopropyl Dibenzoylméthane ;

### Aminobenzophénones :

- 2-(4-diéthylamino-2-hydroxybenzoyl)-benzoate de n-hexyle vendu notamment sous le nom commercial « UVINUL A +» par BASF ;

### Dérivés anthraniliques :

- Menthyl anthranilate vendu notamment sous le nom commercial « NEO HELIOPAN MA » par SYMRISE ;

### Dérivés de 4,4-diarylbutadiène :

- 1,1-dicarboxy (2,2'-diméthyl-propyl)-4,4-diphénylbutadiène ;

Dans le cadre de l'invention, et selon un mode de réalisation particulier, les filtres hydrophobes (A) suivants sont mis en oeuvre dans le kit selon l'invention :
- Butyl Méthoxydibenzoylméthane ; et/ou
- 2-(4-diéthylamino-2-hydroxybenzoyl)-benzoate de n-héxyle ;

Les filtres organiques hydrophobes (A) lorsqu'ils sont présents, sont présents à des teneurs allant de 0,01 à 5% en poids et de préférence de 0,1 à 1% en poids par rapport au poids total des compositions comprises dans le kit de l'invention.

### Filtres hydrophobes (B) capables d'absorber les UV de 280 à 320 nm

### Para-aminobenzoates :

- Ethyl PABA ;
- Ethyl Dihydroxypropyl PABA ;
- Ethylhexyl Diméthyl PABA (ESCALOL 507 de ISP) ;

### Dérivés salicyliques :

- Homosalate vendu notamment sous le nom « Eusolex HMS » par Rona/EM Industries ;
- Ethylhexyl Salicylate vendu notamment sous le nom « NEO HELIOPAN OS » par SYMRISE ;
- Dipropylèneglycol Salicylate vendu notamment sous le nom « DIPSAL » par SCHER ;
TEA Salicylate et sous le nom « NEO HELIOPAN TS » par SYMRISE ;

### Cinnamates

- Ethylhexyl Méthoxycinnamate vendu notamment sous le nom commercial « PARSOL MCX » par DSM Nutritional Products, Inc. ;
- Isopropyl Méthoxy cinnamate ;
- Isoamyl Méthoxy cinnamate vendu notamment sous le nom commercial « NEO HELIOPAN E 1000 » par SYMRISE ;
- Diisopropyl Méthylcinnamate ;
- Cinnoxate ;
- Glycéryl Ethylhexanoate Diméthoxycinnamate ;

### Dérivés de β,β'-diphénylacrylate :

- Octocrylène, vendu notamment sous le nom commercial « UVINUL N539 » par BASF ;
- Etocrylène, vendu notamment sous le nom commercial « UVINUL N35 » par BASF ;

### Dérivés du benzylidène camphre :

- 3-Benzylidène camphre fabriqué sous le nom « MEXORYL SD » par CHIMEX ;
- Méthylbenzylidène camphre vendu notamment sous le nom « EUSOLEX 6300 » par MERCK ;
- Polyacrylamidométhyle Benzylidène Camphre fabriqué sous le nom « MEXORYL SW » par CHIMEX ;

### Dérivés de triazine :

- Ethylhexyl triazone vendu notamment sous le nom commercial « UVINUL T150 » par BASF ;
- Diéthylhexyl Butamido Triazone vendu notamment sous le nom commercial « UVASORB HEB » par SIGMA 3V ;
- 2,4,6-tris(4'-amino benzalmalonate de dinéopentyle)-s-triazine ;
- 2,4,6-tris(4'-amino benzalmalonate de diisobutyle)-s- triazine ;
- 2,4-bis(4'-amino benzalmalonate de dinéopentyle)-6-(4'-aminobenzoate de n-butyle)-s-triazine ;
- 2,4-bis(4'-amino benzoate de n-butyle)-6-(aminopropyltrisiloxane)-s-triazine ;
- les filtres triazines symétriques décrits dans le brevet US 6,225,467, la demande WO 2004/085412 (voir composés 6 et 9) ou le document « Symetrical Triazine Derivatives » IP.COM Journal, IP.COM INC WEST HENRIETTA, NY, US (20 septembre 2004) notamment les 2,4,6-tris-(biphényl)-1,3,5-triazines (en particulier la 2,4,6-tris(biphényl-4-yl-1,3,5-triazine) et la 2,4,6-tris(terphenyl)-1,3,5-triazine, ces deux derniers filtres étant décrits dans les demandes de BEIERSDORF WO 06/035000, WO 06/034982, WO 06/034991, WO 06/035007, WO 2006/034992, WO 2006/034985).

### Dérivés d'imidazolines :

- Ethylhexyl Diméthoxybenzylidene Dioxoimidazoline Propionate,

### Dérivés du benzalmalonate :

- Polyorganosiloxanes à fonction benzalmalonate tels que le Polysilicone-15 vendu notamment sous la dénomination commerciale « PARSOL SLX » par DSM Nutritional Products, Inc. ;
- Di-neopentyl 4'-méthoxybenzalmalonate ;

### Dérivés de mérocyanine :

- Octyl-5-N,N-diéthylamino-2-phénysulfonyl-2,4-pentadiénoate ;

Dans le cadre de l'invention et selon un mode de réalisation particulier, les filtres hydrophobes (B) suivants sont mis en oeuvre dans le kit selon l'invention :
- Homosalate ;
- Ethylhexylsalicylate ;
- Octocrylène ;
- Ethylhexyl Méthoxycinnamate ;
- Isoamyl Méthoxy cinnamate ;
- Ethylhexyl triazone ; et/ou
- Diéthylhexyl Butamido Triazone ;

Selon un mode tout particulier de réalisation, on cite les filtres hydrophobes (B) suivants :
- Ethylhexylsalicylate ;
- Octocrylène ;
- Ethylhexyl triazone ; et/ou
- Ethylhéxyl Méthoxycinnamate ;

Les filtres organiques hydrophobes (B), lorsqu'ils sont présents, sont présents à des teneurs allant de 0,01 à 5% en poids et de préférence de 0,1 à 1% en poids par rapport au poids total des compositions comprises dans le kit de l'invention.

### Filtres hydrophobes (C) mixtes capables d'absorber à la fois les UVA et les UVB

### Dérivés de benzophénone

- Benzophénone-1 vendu notamment sous le nom commercial « UVINUL 400 » par BASF ;
- Benzophénone-2 vendu notamment sous le nom commercial « UVINUL D50 » par BASF ;
- Benzophénone-3 ou Oxybenzone vendu notamment sous le nom commercial « UVINUL M40 » par BASF ;
- Benzophénone-5 ;
- Benzophénone-6 vendu notamment sous le nom commercial « Helisorb 11 » par Norquay ;
- Benzophénone-8 vendu notamment sous le nom commercial « Spectra-Sorb UV-24 » par American Cyanamid ;
- Benzophénone-10 ;
- Benzophénone-11 ;
- Benzophénone-12 ;

### Dérivés du phényl benzotriazole :

- Drométrizole Trisiloxane vendu notamment sous le nom « Silatrizole » par RHODIA CHIMIE ou fabriqué sous le nom « Meroxyl XL » par la société CHIMEX ;
- Méthylène bis-Benzotriazolyl Tétramethylbutylphénol, vendu sous forme solide notamment sous le nom commercial « MIXXIM BB/100 » par FAIRMOUNT CHEMICAL ou sous forme micronisé en dispersion aqueuse notamment sous le nom commercial « TINOSORB M » par CIBA SPECIALTY CHEMICALS ;

### Dérivés bis-résorcinyl triazines

- Bis-Ethylhexyloxyphénol Méthoxyphenyl Triazine vendu notamment sous le nom commercial « TINOSORB S » par CIBA GEIGY ;

### Dérivés de benzoxazole :

- 2,4-bis-[5-1(diméthylpropyl)benzoxazol-2-yl-(4-phenyl)-imino]-6-(2-ethylhexyl)-imino-1,3,5-triazine vendu notamment sous le nom d'Uvasorb K2A par Sigma 3V ;

Dans le cadre de l'invention et selon un mode de réalisation particulier, les filtres hydrophobes (C) suivants sont mis en oeuvre dans le kit selon l'invention :
- Drométrizole Trisiloxane ;
- Méthylène bis-Benzotriazolyl Tétramethylbutylphénol ;
- Bis-Ethylhexyloxyphénol Méthoxyphenyl Triazine ; et/ou
- Benzophenone-3 ou Oxybenzone ;

Selon un mode tout particulier de réalisation, on cite les filtres hydrophobes (C) suivants :
- Drométrizole Trisiloxane ;
- Bis-Ethylhexyloxyphénol Méthoxyphenyl Triazine.

Les filtres organiques hydrophobes (C), lorsqu'ils sont présents, sont présents à des teneurs allant de 0,01 à 5 % en poids et de préférence de 0,1 à 1 % en poids par rapport au poids total des compositions comprises dans le kit de l'invention.

### Filtres solaires ou photoprotecteurs inorganiques (D)

Les agents photoprotecteurs inorganiques (D) sont choisis parmi des pigments d'oxydes métalliques enrobés ou non (taille moyenne des particules primaires: généralement entre 5 nm et 100 nm, de préférence entre 10 nm et 50 nm) comme par exemple des pigments d'oxyde de titane (amorphe ou cristallisé sous forme rutile et/ou anatase), de fer, de zinc, de zirconium ou de cérium qui sont tous des agents photoprotecteurs UV bien connus en soi.

Les pigments peuvent être enrobés ou non enrobés.

Les pigments enrobés sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p. 53-64, tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

De façon connue, les silicones sont des polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane), des radicaux hydrocarbonés éventuellement substitués étant directement liés par l'intermédiaire d'un atome de carbone sur lesdits atomes de silicium.

Le terme "silicones" englobe également les silanes nécessaires à leur préparation, en particulier, les alkyl silanes.

Les silicones utilisées pour l'enrobage des pigments convenant à la présente invention sont de préférence choisies dans le groupe contenant les alkyl silanes, les polydialkylsiloxanes, et les polyalkylhydrogénosiloxanes. Plus préférentiellement encore, les silicones sont choisies dans le groupe contenant l'octyl triméthyl silane, les polydiméthylsiloxanes et les polyméthylhydro-génosiloxanes.

Bien entendu, les pigments d'oxydes métalliques avant leur traitement par des silicones, peuvent avoir été traités par d'autres agents de surface, en particulier par de l'oxyde de cérium, de l'alumine, de la silice, des composés de l'aluminium, des composés du silicium, ou leurs mélanges.

Les pigments enrobés sont plus particulièrement des oxydes de titane enrobés :
- de silice, tels que le produit « SUNVEIL » de la société IKEDA,
- de silice et d'oxyde de fer tels que le produit « SUNVEIL F » de la société IKEDA,
- de silice et d'alumine tels que les produits « MICROTITANIUM DIOXIDE MT 500 SA» et «MICROTITANIUM DIOXIDE MT 100 SA» société TAYCA, « TIOVEIL » de la société TIOXIDE,
- d'alumine tels que les produits « TIPAQUE TTO-55 (B) » et « TIPAQUE TTO-55 (A) » de la société ISHIHARA, et « UVT 14/4 » de la société KEMIRA,
- d'alumine et de stéarate d'aluminium tels que les produits « MICROTITANIUM DIOXIDE MT 100 T, MT 100 TX, MT 100 Z, MT-01 » de la société TAYCA, les produits « Solaveil CT-10 W » et « Solaveil CT 100 » de la société UNIQEMA et le produit « Eusolex T-AVO » de la société MERCK,
- de silice, d'alumine et d'acide alginique tel que le produit « MT-100 AQ » de la société TAYCA,
- d'alumine et de laurate d'aluminium tel que le produit « MICROTITANIUM DIOXIDE MT 100 S » de la société TAYCA,
- d'oxyde de fer et de stéarate de fer tels que le produit « MICROTITANIUM DIOXIDE MT 100 F » de la société TAYCA,
- d'oxyde de zinc et de stéarate de zinc tels que le produit « BR 351 » de la société TAYCA,
- de silice et d'alumine et traités par une silicone tels que les produits « MICROTITANIUM DIOXIDE MT 600 SAS », « MICROTITANIUM DIOXIDE MT 500 SAS » ou « MICROTITANIUM DIOXIDE MT 100 SAS » de la société TAYCA,
- de silice, d'alumine, de stéarate d'aluminium et traités par une silicone tels que le produit « STT-30-DS » de la société TITAN KOGYO,
- de silice et traité par une silicone tel que le produit « UV-TITAN X 195 » de la société KEMIRA,
- d'alumine et traités par une silicone tels que les produits « TIPAQUE TTO-55 (S) » de la société ISHIHARA, ou « UV TITAN M 262 » de la société KEMIRA,
- de triéthanolamine tels que le produit « STT-65-S » de la société TITAN KOGYO,
- d'acide stéarique, tels que le produit « TIPAQUE TTO-55 (C) » de la société ISHIHARA,
- d'hexamétaphosphate de sodium tels que le produit « MICROTITANIUM DIOXIDE MT 150 W » de la société TAYCA.
- le TiO₂ traité par l'octyl triméthyl silane vendu sous la dénomination commerciale « T 805 » par la société DEGUSSA SILICES,
- le TiO₂ traité par un polydiméthylsiloxane vendu sous la dénomination commerciale « 70250 Cardre UF TiO2SI3 » par la société CARDRE,
- le TiO₂ anatase/rutile traité par un polydiméthylhydrogénosiloxane vendu sous la dénomination commerciale « MICRO TITANIUM DIOXYDE USP GRADE HYDROPHOBIC » par la société COLOR TECHNIQUES.

Les pigments d'oxyde de titane non enrobés sont par exemple vendus par la société TAYCA sous les dénominations commerciales « MICROTITANIUM DIOXIDE MT 500 B » ou « MICROTITANIUM DIOXIDE MT600 B », par la société DEGUSSA sous la dénomination « P 25 », par la société WACKHER sous la dénomination « Oxyde de titane transparent PW », par la société MIYOSHI KASEI sous la dénomination « UFTR », par la société TOMEN sous la dénomination « ITS » et par la société TIOXIDE sous la dénomination « TIOVEIL AQ ».

Les pigments d'oxyde de zinc non enrobés, sont par exemple :
- ceux commercialisés sous la dénomination « Z-cote » par la société Sunsmart ;
- ceux commercialisés sous la dénomination « Nanox » par la société Elementis ;
- ceux commercialisés sous la dénomination « Nanogard WCD 2025 » par la société Nanophase Technologies ;

Les pigments d'oxyde de zinc enrobés sont par exemple :
- ceux commercialisés sous la dénomination « Oxide zinc CS-5 » par la société Toshibi (ZnO enrobé par polymethylhydrogenesiloxane) ;
- ceux commercialisés sous la dénomination « Nanogard Zinc Oxide FN » par la société Nanophase Technologies (en dispersion à 40 % dans le Finsolv TN, benzoate d'alcools en C₁₂-C₁₅) ;
- ceux commercialisés sous la dénomination « DAITOPERSION ZN-30 » et « DAITOPERSION Zn-50 » par la société Daito (dispersions dans cyclopolyméthylsiloxane /polydiméthylsiloxane oxyéthyléné, contenant 30 % ou 50 % de nano-oxydes de zinc enrobés par la silice et le polyméthylhydrogènesiloxane) ;
- ceux commercialisés sous la dénomination « NFD Ultrafine ZnO » par la société Daikin (ZnO enrobé par phosphate de perfluoroalkyle et copolymère à base de perfluoroalkyléthyle en dispersion dans du cyclopentasiloxane) ;
- ceux commercialisés sous la dénomination « SPD-Z1 » par la société Shin-Etsu (ZnO enrobé par polymère acrylique greffé silicone, dispersé dans cyclodiméthylsiloxane) ;
- ceux commercialisés sous la dénomination « Escalol Z100 » par la société ISP (ZnO traité alumine et dispersé dans le mélange méthoxycinnamate d'éthylhexyle/copolymère PVP-hexadécene/méthicone) ;
- ceux commercialisés sous la dénomination «Fuji ZnO-SMS-10» par la société Fuji Pigment (ZnO enrobé silice et polyméthylsilsesquioxane) ;
- ceux commercialisés sous la dénomination « Nanox Gel TN » par la société Elementis (ZnO dispersé à 55 % dans du benzoate d'alcools en C₁₂-C₁₅ avec polycondensat d'acide hydroxystéarique).

Un pigment d'oxyde de cérium non enrobé est, par exemple, vendu sous la dénomination « COLLOIDAL CERIUM OXIDE » par la société RHONE POULENC.

Les pigments d'oxyde de fer non enrobés sont par exemple vendus par la société ARNAUD sous les dénominations « NANOGARD WCD 2002 (FE 45B) », « NANOGARD IRON FE 45 BL AQ », « NANOGARD FE 45R AQ », « NANOGARD WCD 2006 (FE 45R) », ou par la société MITSUBISHI sous la dénomination « TY-220 ».

Les pigments d'oxyde de fer enrobés sont par exemple vendus par la société ARNAUD sous les dénominations «NANOGARD WCD 2008 (FE 45B FN) », « NANOGARD WCD 2009 (FE 45B 556) », « NANOGARD FE 45 BL 345 », « NANOGARD FE 45 BL », ou par la société BASF sous la dénomination « OXYDE DE FER TRANSPARENT ».

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination « SUNVEIL A », ainsi que le mélange de dioxyde de titane et de dioxyde de zinc enrobé d'alumine, de silice et de silicone tel que le produit « M 261 » vendu par la société KEMIRA ou enrobé d'alumine, de silice et de glycérine tel que le produit « M 211 » vendu par la société KEMIRA.

Dans le cadre de l'invention et selon le mode de réalisation particulier, les filtres organiques (D) suivants sont mis en oeuvre dans le kit selon l'invention : oxyde de titane amorphe ou cristallisé sous forme rutile et/ou anatase.

Les filtres inorganiques (D), lorsqu'ils sont présents, sont présents à des teneurs allant de 0,01 à 5 % en poids et de préférence de 0,1 à 1 % en poids par rapport au poids total des compositions comprises dans le kit de l'invention.

La teneur totale en filtres solaires (A), (B), (C) et (D) est, selon un mode de réalisation particulier, inférieure ou égale à 15 % en poids, particulièrement va de 0,01 à 10 % en poids, plus particulièrement de 0,1 à 5 % en poids, par rapport au poids total de l'ensemble des compositions dans ledit kit.

Selon un mode de réalisation particulier, le kit comprend au moins un filtre solaire (A) anti UVA, organique et hydrophobe, et au moins un filtre solaire (B) anti UVB, organique et hydrophobe et éventuellement un filtre minéral (D).

Selon un autre mode de réalisation particulier, le kit comprend au moins un filtre solaire (C) anti UVA et anti UVB, organique et hydrophobe, et éventuellement un filtre minéral (D).

Selon encore un autre mode de réalisation particulier, le kit comprend au moins un filtre solaire (A) anti UVA, organique et hydrophobe, et au moins un filtre minéral (D).

Selon encore un autre mode de réalisation de l'invention, le kit comprend au moins du Butyl Méthoxydibenzoylméthane vendu notamment sous le nom commercial « PARSOL 1789 » et de l'Ethylhexyl Méthoxycinnamate vendu notamment sous le nom commercial « PARSOL MCX ».

Selon encore un autre mode de réalisation de l'invention, le kit comprend au moins du Butyl Méthoxydibenzoylméthane vendu notamment sous le nom commercial « PARSOL 1789 » et au moins un dioxyde de titane enrobé ou non comme filtre minéral.

Selon encore un autre mode de réalisation de l'invention, le kit comprend au moins du Butyl Methoxydibenzoylmethane vendu notamment sous le nom commercial « PARSOL 1789 » et au moins de l'Ethylhexyl triazone vendu notamment sous le nom commercial « UVINUL T150 » par BASF.

Selon encore un autre mode de réalisation de l'invention, le kit comprend au moins du 2-(4-diéthylamino-2-hydroxybenzoyl)-benzoate de n-hexyle vendu notamment sous le nom commercial « UVINUL A+ » par BASF et au moins du éthylhexyl Méthoxycinnamate vendu notamment sous le nom commercial « PARSOL MCX » par DSM Nutritional Products, Inc..

Selon encore un autre mode de réalisation de l'invention, le kit comprend au moins du Butyl Méthoxydibenzoylméthane vendu notamment sous le nom commercial « PARSOL 1789 » et au moins de l'Octocrylène, vendu notamment sous le nom commercial « UVINUL N539 » par BASF.

Selon encore un autre mode de réalisation de l'invention, le kit comprend au moins du Drométrizole Trisiloxane vendu sous le nom « Silatrizole » par RHODIA CHIMIE.

Selon encore un autre mode de réalisation de l'invention, le kit comprend au moins du Bis-Ethylhexyloxyphénol Méthoxyphényl Triazine vendu sous le nom commercial « TINOSORB S » par CIBA GEIGY.

Selon un mode de réalisation, le kit comprend au moins deux compositions différentes conditionnées séparément, la première composition contenant, dans un milieu physiologiquement acceptable, au moins un composé (X), la deuxième composition contenant, dans un milieu physiologiquement acceptable, au moins un composé (Y), et l'éventuel catalyseur ou l'éventuel peroxyde étant présent dans l'une ou l'autre desdites première et deuxième compositions, et/ou présent éventuellement dans une autre composition.

Le kit selon l'invention peut éventuellement comprendre, en outre, au moins un filtre photoprotecteur choisi parmi les filtres solaires anti UV A et/ou anti UV B organiques et hydrophiles.

Outre les composés requis décrits ci-dessus, le kit selon l'invention peut contenir un milieu physiologiquement acceptable tel que défini ci-après.

Il peut s'agir plus particulièrement desdites première et/ou seconde compositions du kit selon l'invention qui peuvent comprendre, en outre, au moins un milieu physiologiquement acceptable.

Selon un mode de réalisation, ladite composition (A1) du kit selon l'invention comprend en outre au moins un milieu physiologiquement acceptable et/ou ladite composition (A2) du kit selon l'invention comprend en outre au moins un milieu physiologiquement acceptable.

Selon un autre mode de réalisation, ladite composition (A3) du kit selon l'invention comprend en outre au moins un milieu physiologiquement acceptable.

### MILIEU PHYSIOLOGIQUEMENT ACCEPTABLE

Comme précisé précédemment, les compositions du kit selon l'invention comprennent un milieu physiologiquement acceptable, c'est à dire un milieu non toxique et susceptible d'être appliqué sur les matières kératiniques d'êtres humains et d'aspect, d'odeur et de toucher agréables.

Les compositions du kit selon l'invention contiennent en général essentiellement les ingrédients (X), (Y), (A), (B), (C) et/ou (D), ainsi que le catalyseur et/ou le péroxyde tels que définis précédemment.

Ainsi, selon un mode de réalisation particulier de l'invention, le kit contient jusqu'à 90 % en poids des ingrédients cités ci-dessus, voire jusqu'à 95 %, 99 % ou même 100 % par rapport au poids total de l'ensemble des compositions du kit.

Les compositions du kit selon l'invention sont avantageusement sous la forme anhydre ou essentiellement anhydre et peuvent se présenter sous la forme de liquides, de pâtes souples.

On parle de composition « anhydre » lorsque la teneur en eau et/ou solvant(s) hydrosoluble(s) dans la composition est inférieure à 3 % en poids par rapport au poids total de la composition, typiquement inférieur à 1,5 %, 1 % voire 0,5 %.

Les compositions de ce type peuvent avoir la forme d'un produit de soin ou de maquillage du visage et/ou du corps, et être conditionnées par exemple sous forme de crème en pot ou de fluide en tube ou en flacon pompe.

Les compositions de l'invention peuvent comprendre en plus une ou plusieurs huiles permettant de solubiliser au moins un des ingrédients (X), (Y), (A), (B), (C) et (D) de l'invention.

Comme exemples d'huiles utilisables dans les compositions du kit selon l'invention, on peut citer :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules R₁COOR₂ et R₁OR₂ dans laquelle R₁ représente le reste d'un acide gras comportant de 8 à 29 atomes de carbone, et R₂ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, l'isohexadecane, l'isododecane, la vaseline, les polydécènes, le polyisobutène hydrogéné tel que l'huile de Parléam^{®} ;
- des huiles essentielles naturelles ou synthétiques telles que, par exemple, les huiles d'eucalyptus, de lavandin, de lavande, de vétiver, de litsea cubeba, de citron, de santal, de romarin, de camomille, de sarriette, de noix de muscade, de cannelle, d'hysope, de carvi, d'orange, de géraniol, de cade et de bergamote;
- les alcools gras ayant de 8 à 26 atomes de carbone, comme l'alcool cétylique, l'alcool stéarylique et leur mélange (alcool cétylstéarylique), l'octyl dodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que le cyclohexasiloxane et le cyclopentasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les gommes telles que les gommes de silicone (diméthiconol).

Ces corps gras peuvent être choisis de manière variée par l'homme du métier afin de préparer une composition ayant les propriétés, par exemple de consistance ou de texture souhaitée.

Les compositions du kit selon l'invention peuvent comprendre au moins une huile volatile.

Par « huile volatile », on entend au sens de l'invention une huile susceptible de s'évaporer au contact des matières kératiniques en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant en particulier de 0,13 Pa à 40 000 Pa (10-3 à 300 mm de Hg), en particulier allant de 1,3 Pa à 13 000 Pa (0,01 à 100 mm de Hg), et plus particulièrement allant de 1 ,3 Pa à 1300 Pa (0,01 à 10 mm de Hg).

On peut citer comme huiles volatiles entre autres, les silicones cycliques ou linéaires renfermant de 2 à 6 atomes de silicium, telles que le cyclohexasiloxane, le dodécaméthylpentasiloxane, le décaméthyltétrasiloxane, le butyltrisiloxane et l'éthyltrisiloxane. On peut aussi utiliser les hydrocarbures ramifiés tels que par exemple l'isododécane ainsi que les perfluoroalcanes volatiles tels que le dodécafluoropentane et le tétradécafluorohexane, vendus sous les dénominations de « PF 5050^{®} » et « PF 5060^{®} » par la Société 3M et les dérivés de perfluoromorpholine, tels que la 4-trifluorométhyl perfluoromorpholine vendue sous la dénomination « PF 5052^{®} » par la Société 3M.

La quantité de phase huileuse présente dans les compositions du kit selon l'invention peut aller par exemple de 0,01 à 30 % en poids et de préférence de 0,1 à 10 % en poids par rapport au poids total de la composition contenant une telle phase huileuse.

Les compositions du kit selon l'invention peuvent, en outre, comprendre au moins une matière colorante choisie par exemple parmi les pigments, les nacres, les colorants, les matériaux à effet et leurs mélanges.

Ces matières colorantes peuvent être présentes en une teneur allant de 0,01 % à 10 % en poids, de préférence de 0,01 % à 5 % par rapport au poids total de l'ensemble des compositions comprises dans le kit selon l'invention.

Les compositions du kit selon l'invention peuvent comprendre une charge notamment en une teneur allant de 0,01 % à 10 % en poids, par rapport au poids total de l'ensemble des compositions comprises dans le kit selon l'invention de préférence allant de 0,01 % à 5 % en poids. Ces charges peuvent être minérales ou organiques de toute forme, plaquettaires, sphériques ou oblongues, quelle que soit la forme cristallographique (par exemple feuillet, cubique, hexagonale, orthorombique, ou amorphe). On peut citer la silice, le talc, le mica, le kaolin, la lauroyl-lysine, l'amidon, le nitrure de bore, les poudres de PTFE, les poudres de PMMA, les poudres de résine de méthyl silsesquioxane (comme le Tospearl 145A de GE Silicone) , les particules hémisphérique creuse de résine de silicone (comme les NLK 500, NLK 506 et NLK 510 de Takemoto Oil and Fat ), le sulfate de Baryum, le carbonate de calcium précipité, le carbonate et l'hydro-carbonate de magnésium, l'hydroxyapatite, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les compositions du kit selon l'invention peuvent en outre contenir divers adjuvants couramment utilisés dans le domaine cosmétique, tels que des séquestrants ; des parfums ; des épaississants et gélifiants lipophiles. Les quantités de ces différents adjuvants et leur nature seront choisies de manière à ne pas nuire à la formation du film photoprotecteur ni aux propriétés avantageuses desdits films photoprotecteurs.

Selon la fluidité de la composition que l'on souhaite obtenir, on peut incorporer dans la composition, un ou plusieurs gélifiants lipophiles, c'est-à-dire solubles ou dispersibles dans les huiles.

Comme gélifiants lipophiles, on peut citer par exemple les argiles modifiées telles que le silicate de magnésium modifié (bentone gel VS38 de RHEOX), l'hectorite modifiée par le chlorure de distéaryl diméthyl ammonium (nom CTFA : Disteardimonium hectorite) commercialisé sous la dénomination « bentone 38 CE » par la société RHEOX.

Pour une application en particulier sur des peaux grasses, les compositions du kit selon l'invention peuvent comprendre au moins un actif choisi parmi : les agents desquamants, les agents anti-séborrhéiques, les agents anti-microbiens, et les agents apaisants.

Pour une application en particulier sur des peaux âgées, les compositions du kit selon l'invention peuvent comprendre au moins un actif choisi parmi : les agents desquamants, les agents dépigmentants ou anti-pigmentants, les agents anti-glycation, les agents anti-NO, les agents stimulant la synthèse de macromolécules dermiques ou épidermiques et/ou empêchant leur dégradation, les agents stimulant la prolifération des fibroblastes ou des kératinocytes et/ou la différenciation des kératinocytes, les agents myorelaxants ou dermo-décontractants, les agents anti-radicalaires ou anti-pollution, les agents tenseurs, les agents agissant sur la micro-circulation.

Pour une application, en particulier sur une cicatrice, les kits selon l'invention peuvent comprendre au moins un agent favorisant la cicatrisation comme par exemple les extraits de *Centella Asiatica* comme le MADECASSOSIDE vendu par la société BAYER.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour chaque composition du kit selon l'invention.

Bien entendu, l'homme du métier veillera à choisir le ou les éventuel(s) additif(s) complémentaire(s) et/ou leur quantité de telle manière que la formation du film photoprotecteur et les propriétés avantageuses desdits films résultants ne soient pas, ou substantiellement pas, altérées par l'adjonction envisagée.

### CONDITIONNEMENT

Les premières et secondes compositions ci-dessus peuvent être conditionnées dans des conditionnements différents, indépendants, ou en variante dans un même dispositif de conditionnement comportant deux compartiments pouvant ou non être mis extemporanément en communication.

De préférence, la première composition comprenant, par exemple, le composé (X) et la seconde composition comprenant, par exemple, le composé (Y) sont conditionnées dans des conditionnements séparés d'un même article de conditionnement.

Chaque composition peut aussi être conditionnée dans un compartiment différent au sein d'un même article conditionnement, le mélange des deux compositions s'effectuant par exemple à la ou aux extrémités de l'article de conditionnement lors de la délivrance de chaque composition.

Dans un autre exemple, chacune des compositions ci-dessus est contenue dans un compartiment respectif d'un dispositif de conditionnement, les compartiments étant fermés par un obturateur pouvant passer d'un état d'obturation à un état de non-obturation en réponse à une action de l'utilisateur sur le dispositif de conditionnement, par exemple une rotation ou le déplacement d'une partie du dispositif.

Alternativement, chacune des première et seconde compositions peut être conditionnée dans un article de conditionnement différent.

Les première et seconde compositions sont différentes l'une de l'autre.

Par exemple, la première composition est avantageusement dénuée de composé (Y) et la seconde composition est avantageusement dénuée de composé (X). En effet, au regard de leur grande réactivité l'un pour l'autre, les composés (X) et (Y) ne sont pas présents simultanément dans une première et/ou seconde composition formant un kit selon l'invention lorsque leur interaction n'est pas conditionnée par la présence d'un catalyseur ou d'un peroxyde.

Le ou les catalyseurs et/ou peroxydes, ainsi que les filtres solaires (A), (B), (C), et/ou (D) peuvent être présents dans l'une ou l'autre des compositions, en fonction de la compatibilité avec le reste des ingrédients, voire être conditionnés séparément des première et deuxième compositions.

Le cas échéant, le ou les catalyseurs et/ou peroxydes, ainsi que les filtres solaires (A), (B), (C) et/ou (D) peuvent être contenus dans un compartiment d'un dispositif de conditionnement contenant l'une des deux compositions précitées, ce compartiment pouvant être mis extemporanément en communication avec celui contenant l'autre composition.

Les compositions peuvent être conditionnées dans une quantité correspondant à un usage unique après mélange de celles-ci. En variante, les compositions peuvent être conditionnées chacune dans une quantité convenant à plusieurs applications successives.

Le cas échéant, les deux compositions peuvent être extraites de deux récipients ou compartiments respectifs et passer au travers d'un mélangeur avant l'application sur les matières kératiniques.

Les compositions ci-dessus peuvent être conditionnées dans des récipients avec ou sans reprise d'air, en fonction de la conservation souhaitée.

L'invention concerne également un procédé de photoprotection des matières kératiniques, en particulier la peau, contre un rayonnement UV dans la gamme au-dessus de 280 nm couvrant les régions UVA et UVB, utilisation d'un kit tel que défini ci-dessus et comprenant l'application sur lesdites matières:
a) d'au moins un composé (X), tel que défini ci-dessus,
b) d'au moins un composé (Y), tel que défini ci-dessus, et
c) d'au moins un système filtrant hydrophobe comprenant :
   (i) au moins un filtre solaire organique hydrophobe (A) capable d'absorber les rayonnements UV de 320 à 400 nm et au moins un filtre solaire organique hydrophobe (B) capable d'absorber les rayonnements UV de 280 à 320 nm et éventuellement au moins un filtre minéral (D) ;
   (ii) au moins un filtre solaire organique hydrophobe (C) capable d'absorber simultanément les rayonnements UV de 280 à 320 nm et 320 à 400 nm, et éventuellement un filtre minéral (D) ; ou
   (iii) au moins un filtre solaire organique hydrophobe (A) capable d'absorber les rayonnements UV de 320 à 400 nm et au moins soit un filtre solaire organique hydrophobe (C) capable d'absorber simultanément les rayonnements UV de 280 à 320 nm et 320 à 400 nm, soit un filtre minéral (D) ;
les applications a), b) et c) ayant lieu soit (i) simultanément par mélange extemporané préalable, soit (ii) par mélange au moment de leur application de façon simultanée ou séquencée selon un ordre indifférent, sous réserve que les conditions dudit mélange soient propices à la réaction entre lesdits composés (X) et (Y).

A l'issue de son application sur la peau, un film se forme qui est séché. Ce séchage peut être réalisé à température ambiante ou, le cas échéant, par application d'un mode de séchage. Ce séchage est alors réalisé dans des conditions propices (temps de séchage, température de séchage) pour permettre la formation d'un film photoprotecteur adhérant à la surface de la peau.

Selon un mode de réalisation, le procédé est tel que la teneur totale en filtres solaires (A), (B), (C) et (D) est inférieure ou égale à 15 % en poids, particulièrement de 0,01 à 10 % en poids, plus particulièrement de 0,01 à 3% en poids, par rapport au poids total de l'ensemble des compositions dans ledit kit.

Ce procédé donne lieu à la formation d'un film de photoprotection selon l'invention dans lequel les filtres solaires sont tels que définis plus haut.

Ce film peut être transparent et peut présenter une épaisseur allant de 50 à 500 µm, de préférence allant de 100 à 200 µm.

Les exemples qui suivent sont présentés à titre illustratif et non limitatif de l'invention. Les pourcentages sont exprimés en poids par rapport au poids total de la composition considérée.

### Exemple 1 : Kit de protection solaire

• Le kit selon l'invention comprend les compositions (A1) et (A2) suivantes, conformes à l'invention :

| Composition | Ingrédients | Teneur (% en poids) |
|---|---|---|
| Composition (A1) | DOW CORNING 7-FC4210 CURING AGENT^{®} | 100 |
| Composition (A2) | DOW CORNING 7-FC4210 ELASTOMER FILM FORMING BASE^{®} | 91,5 |
| | BUTYL METHOXYDIBENZOYL METHANE (1) | 0,5 |
| | DROMETRIZOLE TRISILOXANE (2) | 3 |
| | ALKYLBENZOATE EN C12-C15 (3) | 5 |

| | | |
|---|---|---|
| (1) Parsol^{®} 1789 de la société DSM Nutritional Products, Inc, (2) Silatrizole^{®} de la société RHODIA CHIMIE (3) Finsolv^{®} TN de la société. FINNETEX | | |

Le mélange DOW CORNING 7-FC4210 ELASTOMER FILM-FORMING BASE^{®} commercialisé en « prêt à l'emploi » correspondant à la composition suivante :

| Ingrédient (Nom INCI) | N°CAS | Teneur (% en poids) | Fonction |
|---|---|---|---|
| Dimethyl Siloxane, Dimethylvinylsiloxy-terminated | 68083-19-2 | 55-95 | Polymère |
| Silica Silylate | 68909-20-6 | 10-40 | Charge |
| 1,3-Diethenyl-1,1,3,3-Tetramethyldisiloxane complexes | 68478-92-2 | Trace | Catalyseur |
| Tetramethyldivinyldisiloxane | 2627-95-4 | 0,1-1 | Polymère |

Le mélange DOW CORNING 7-FC4210 CURING AGENT^{®} commercialisé en « prêt à l'emploi » correspondant à la composition suivante:

| Ingrédient (Nom INCI) | N°CAS | Teneur (% en poids) | Fonction |
|---|---|---|---|
| Dimethyl Siloxane, Dimethylvinylsiloxy-terminated | 68083-19-2 | 55-95 | Polymère |
| Silica Silylate | 68909-20-6 | 10-40 | Charge |
| Dimethyl, Methylhydrogen Siloxane, trimethylsiloxy-terminated | 68037-59-2 | 1-10 | Polymère |

Mode opératoire : on mélange tous les composés de (A2) et on obtient une composition (A2).

### • Procédé de photoprotection selon l'invention

La composition (A1) est appliquée sur une zone de peau d'un individu destinée à être protégée du soleil. La composition (A2) est ensuite à son tour appliquée sur cette même zone et les deux compositions (A1) et (A2) sont intimement mélangées par un léger massage sur la zone cutanée concernée. Il se forme alors un film par réaction entre les deux compositions. Ce film, après séchage à l'air libre adhère à la peau. Il pourra être retiré ultérieurement à la façon d'un patch.

### • Evaluation de l'efficacité du film selon l'invention

L'efficacité du kit selon l'invention a été évaluée en mesurant le SPF du film obtenu à l'issue du mélange des compositions (A1) et (A2) ci-dessus appliquées avec un tire-film 120 µm sur une plaque de quartz dépoli.

Le SPF de ce film est mesuré à l'aide d'un spectroradiomètre labsphere UV 1000 S en utilisant la méthode *in vitro* décrite par V. Wandel et al. dans SOSW Journal 127 (2001), modifié en ce qui concerne l'application du produit.

Le SPF est mesuré en 5 points de la plaque et une moyenne est effectuée. On obtient un SPF de 46 ± 0,9.

### Exemple 2 : Kit de protection solaire

• Le kit selon l'invention comprend les compositions (A1) et (A2) suivantes, conformes à l'invention.

| Composition | Ingrédients | Teneur (% en poids) |
|---|---|---|
| Composition (A1) | DOW CORNING 7-FC4210 CURING AGENT^{®} | 100 |
| Composition (A2) | DOW CORNING 7-FC4210 ELASTOMER FILM FORMING BASE^{®} | 96 |
| | TITANIUM DIOXIDE (and) ISOHEXADECANE (and) TRIETHYLHEXANOIN (and) ALUMINUM STEARATE (and) ALUMINA (and) POLYHYDROXYSTEARIC ACID (4) | 2,5 |
| | DROMETRIZOLE TRISILOXANE (2) | 1,5 |

| | | |
|---|---|---|
| (2) Silatrizole^{®} de la société RHODIA CHIMIE (4) Solaveil^{®} CT200 de la société CRODA à 39% en dispersion huileuse avec 1 % TiO₂ | | |

Mode opératoire : on mélange tous les composés de (A2) et on obtient une composition (A2).

### • Procédé protection selon l'invention

La composition (A2) est appliquée sur une zone de peau d'un individu destinée à être protégée du soleil. La composition (A1) est ensuite à son tour appliquée sur cette même zone et les deux compositions (A1) et (A2) sont intimement mélangées par un léger massage sur la zone cutanée concernée. Il se forme alors un film par réaction entre les deux compositions. Ce film, après séchage adhère à la peau. Il pourra être retiré ultérieurement à la façon d'un patch.

### • Evaluation de l'efficacité du film selon l'invention

L'efficacité du kit selon l'invention a été évaluée en mesurant le SPF du film obtenu à l'issue du mélange des compositions (A1) et (A2) ci-dessus appliquées avec un tire-film 120 µm sur une plaque de quartz dépoli.

Le SPF de ce film est mesuré à l'aide d'un spectroradiomètre labsphère UV 1000 S en utilisant la méthode *in vitro* décrite par V. Wandel et al. dans SOFW Journal 127 (2001), modifié en ce qui concerne l'application du produit.

Le SPF est mesuré en 5 points de la plaque et une moyenne est effectuée. On obtient un SPF de 97,2 ± 0,9.

### Exemple 3 : (Comparatif): Kit de protection solaire

### • Le kit contraire à l'invention comprend les compositions (A1) et (A2) suivantes :

| Composition | Ingrédients | Teneur (% en poids) |
|---|---|---|
| Composition (A1) | DOW CORNING 7-FC4210 CURING AGENT^{®} | 100 |
| Composition (A2) | DOW CORNING 7-FC4210 ELASTOMER film forming base^{®} | 96,5 |
| | ETHYLHEXYL METHOXYCINNAMATE (5) | 3,5 |

| | | |
|---|---|---|
| (5) Parsol^{®} MCX de la société DSM NUTRITIONAL PRODUCTS, INC, | | |

### • Procédé protection selon l'invention

La composition (A2) est appliquée sur une zone de peau d'un individu destinée à être protégée du soleil. La composition (A1) est ensuite à son tour appliquée sur cette même zone et les deux compositions (A1) et (A2) sont intimement mélangées par un léger massage sur la zone cutanée concernée. Il se forme alors un film par réaction entre les deux compositions. Ce film, après séchage adhère à la peau. Il pourra être retiré ultérieurement à la façon d'un patch.

### • Evaluation de l'efficacité du film hors invention

L'efficacité du kit selon l'invention a été évaluée en mesurant le SPF du film obtenu à l'issue du mélange des compositions (A1) et (A2) ci-dessus appliquées avec un tire-film 120 µm sur une plaque de quartz dépoli.

Le SPF de ce film est mesuré à l'aide d'un spectroradiomètre labsphère UV 1000 S en utilisant la méthode *in vitro* décrite par V. Wandel et al. dans SOFW Journal 127 (2001), modifié en ce qui concerne l'application du produit.

Le SPF est mesuré en 5 points de la plaque et une moyenne est effectuée. On obtient un SPF de 17,2 ± 0,04.

### Exemples 4 et 5 : Kits de protection solaire

| | | 4 (comparatif) | 5 (invention) |
|---|---|---|---|
| (A1) | DOW CORNING 7-FC4210 CURING AGENT^{®} | 20 | 20 |
| (A2) | DOW CORNING 7-FC4210 ELASTOMER FILM FORMING BASE^{®} | 73 | 73 |
| | BENZOATE D'ALCOOLS C12/C15 (2) | 5 | 5 |
| | BUTYL METHOXYDIBENZOYL METHANE (3) | - | 1 |
| | ETHYLHEXYL TRIAZONE (4) | 2 | 1 |

| | | | |
|---|---|---|---|
| (2) FINSOLV^{®} TN de la société. FINNETEX (3) PARSOL^{®} 1789 de la société DSM NUTRITIONAL PRODUCTS, INC, (4) UVINUL T150^{®}de la société BASF | | | |

### • Procédé protection selon l'invention

Les compositions (A1) et (A2) sont mélangées extemporanément puis appliquées sur une zone de peau d'un individu destinée à être protégée du soleil. Par un léger massage sur la zone cutanée concernée, il se forme alors un film par réaction entre les deux compositions. Ce film, après séchage adhère à la peau. Il pourra être retiré ultérieurement à la façon d'un patch.

### • Evaluation de l'efficacité des films

L'efficacité des kits a été évaluée en mesurant le SPF de chaque film obtenu à l'issue du mélange des compositions (A1) et (A2) ci-dessus appliquées avec un tire-film 120 µm sur une plaque de quartz dépoli.

Le SPF de ce film est mesuré à l'aide d'un spectroradiomètre labsphère UV 1000 S en utilisant la méthode *in vitro* décrite par V. Wandel et al. dans SOFW Journal 127 (2001), modifié en ce qui concerne l'application du produit.

Les résultats sont indiqués dans le tableau suivant :

| Kit | SPF |
|---|---|
| 4 (comparatif) | |
| Ethylhexyl Triazone (UVB) | 52,7 |

| 5 (invention) | |
|---|---|
| Ethylhexyl Triazone (UVB) | 354,9 |
| + Butyl Méthoxydibenzoylméthane (UVA) | |

On observe dans le kit 5 de l'invention une forte augmentation du SPF avec le mélange filtre UVB hydrophobe (Ethylhexyl Triazone) et filtre UVA hydrophobe (Butyl Méthoxydibenzoylméthane) par rapport au filtre UVB utilisé seul pour une concentration totale en filtre(s) constante (2 %).

### Exemples 6 et 7 : Kits de protection solaire

| Composition | Ingrédients | Teneur en % en poids | |
|---|---|---|---|
| | | 6 (comparatif) | 7 (invention) |
| (A1) | DOW CORNING 7-FC4210 CURING AGENT^{®} | 50,0 | 50,0 |
| (A2) | DOW CORNING 7-FC4210 ELASTOMER FILM FORMING BASE^{®} | 47,5 | 47,0 |
| | BENZOATE D'ALCOOLS C12/C15 (2) | - | 0,5 |
| | ETHYLHEXYL METHOXYCINNAMATE (1) | 2,5 | 1,25 |
| | BUTYL METHOXYDIBENZOYL METHANE (3) | 0 | 1,25 |

| | | | |
|---|---|---|---|
| (1) PARSOL^{®} MCX de la société DSM NUTRITIONAL PRODUCTS, INC, (2) FINSOLV^{®} TN de la société FINNETEX (3) PARSOL^{®} 1789 de la société DSM NUTRITIONAL PRODUCTS, INC | | | |

### • Procédé protection selon l'invention

Les compositions (A1) et (A2) sont mélangées extemporanément puis appliquées sur une zone de peau d'un individu destinée à être protégée du soleil. Par un léger massage sur la zone cutanée concernée, il se forme alors un film par réaction entre les deux compositions. Ce film, après séchage adhère à la peau. Il pourra être retiré ultérieurement à la façon d'un patch.

### • Evaluation de l'efficacité des films

L'efficacité de chaque kit a été évaluée en mesurant le SPF de chaque film obtenu à l'issue du mélange des compositions (A1) et (A2) ci-dessus appliquées avec un tire-film 120 µm sur une plaque de quartz dépoli.

Le SPF de ce film est mesuré à l'aide d'un spectroradiomètre labsphère UV 1000 S en utilisant la méthode *in vitro* décrite par V. Wandel et al. dans SOFW Journal 127 (2001), modifié en ce qui concerne l'application du produit.

Les résultats sont indiqués dans le tableau suivant :

| Kit | SPF |
|---|---|
| 6 (comparatif) | |
| Ethylhexyl Méthoxycinnamate (UVB) | 22 |

| 7 (invention) | |
|---|---|
| Ethylhexyl Méthoxycinnamate (UVB) | 285,5 |
| + Butyl Méthoxydibenzoylméthane (UVA) | |

On observe dans le kit 7 de l'invention une forte augmentation du SPF avec le mélange filtre UVB hydrophobe (Ethylhexyl Triazone) et le filtre UVA hydrophobe (Butyl Méthoxydibenzoylméthane) par rapport au filtre UVB hydrophobe seul pour une concentration totale en filtre(s) constante (2,5 %).

### Exemples 8 à 10 : Kits de protection solaire

| Composition | Ingrédients | Teneur en % en poids | | |
|---|---|---|---|---|
| | | 8 (comparatif) | 9 (comparatif) | 10 (invention) |
| (A1) | DOW CORNING 7-FC4210 CURING AGENT^{®} | 50,0 | 50,0 | 50,0 |
| (A2) | DOW CORNING 7-FC4210 ELASTOMER FILM FORMING BASE^{®} | 45 | 45 | 45 |
| | BENZOATE D'ALCOOLS C12/C15 (2) | - | 4 | 4 |
| | TITANIUM DIOXIDE (and) ISOHEXADECANE (and) TRIETHYLHEXANOIN (and) ALUMINUM STEARATE (and) ALUMINA (and) POLYHYDROXYSTEARIC ACID (4) | 1MA* | - | 0,5 MA* |
| | BUTYL METHOXYDIBENZOYL METHANE (3) | - | 1 | 0,5 |

| | | | | |
|---|---|---|---|---|
| * Matière active (2) FINSOLV^{®} TN de la société. FINNETEX (3) PARSOL^{®} 1789 de la société DSM NUTRITIONAL PRODUCTS, INC, (4) Solaveil^{®} CT200 de la société CRODA.à 39% en dispersion huileuse avec 1 % TiO2 | | | | |

### • Procédé protection selon l'invention

Les compositions (A1) et (A2) sont mélangées extemporanément puis appliquées sur une zone de peau d'un individu destinée à être protégée du soleil. Par un léger massage sur la zone cutanée concernée, il se forme alors un film par réaction entre les deux compositions. Ce film, après séchage adhère à la peau. Il pourra être retiré ultérieurement à la façon d'un patch.

### • Evaluation de l'efficacité des films

L'efficacité de chaque kit a été évaluée en mesurant le SPF de chaque film obtenu à l'issue du mélange des compositions (A1) et (A2) ci-dessus appliquées avec un tire-film 120 µm sur une plaque de quartz dépoli.

Le SPF de ce film est mesuré à l'aide d'un spectroradiomètre labsphère UV 1000 S en utilisant la méthode *in vitro* décrite par V. Wandel et al. dans SOFW Journal 127 (2001), modifié en ce qui concerne l'application du produit.

Les résultats sont indiqués dans le tableau suivant

| Kit | SPF |
|---|---|
| 8 (comparatif) | |
| | 1,4 |
| TiO₂ (Filtre minéral) | |
| 9 (comparatif) | |
| | |
| Butyl | 6,6 |
| Méthoxydibenzoylméthane | |
| (UVA) | |
| 10 (invention) | |
| | |
| TiO₂ (Filtre minéral) + | |
| | 36,9 |
| Butyl | |
| Méthoxydibenzoylméthane | |
| (UVA) | |

On observe dans le kit 10 de l'invention une forte augmentation du SPF avec le mélange filtre minéral (TiO₂) et filtre UVA hydrophobe (Butyl Méthoxydibenzoylméthane) par rapport au filtre minéral seul et au filtre UVA seul pour une concentration totale en filtre(s) constante (1 %).

## Revendications

1. Kit de photoprotection des matières kératiniques contre un rayonnement UV dans la gamme au dessus de 280 nm couvrant les UV A et les UV B, comprenant au moins deux compositions différentes conditionnées séparément, ledit kit comprenant :
• au moins un composé (X),
• au moins un composé (Y),
• éventuellement au moins un catalyseur ou au moins un peroxyde, et
• au moins un système filtrant hydrophobe comprenant :
(i) au moins un filtre solaire organique hydrophobe (A) capable d'absorber les rayonnements UV de 320 à 400 nm et au moins un filtre solaire organique hydrophobe (B) capable d'absorber les rayonnements UV de 280 à 320 nm, et éventuellement un filtre minéral (D) ;
(ii) au moins un filtre solaire organique hydrophobe (C) capable d'absorber simultanément les rayonnements UV de 280 à 320 nm et 320 à 400 nm, et éventuellement un filtre minéral (D) ; ou
(iii) au moins un filtre solaire organique hydrophobe (A) capable d'absorber les rayonnements UV de 320 à 400 nm et au moins soit un filtre solaire organique hydrophobe (C) capable d'absorber simultanément les rayonnements UV de 280 à 320 nm et 320 à 400 nm, soit un filtre minéral (D) ;
l'un au moins desdits composés (X) et (Y) étant siliconé et à la condition que les composés (X), (Y) et éventuellement le catalyseur ou le peroxyde ne sont pas présents simultanément dans la même composition, lesdits composés (X) et (Y) étant susceptibles de réagir ensemble par une réaction d'hydrosilylation en présence dudit catalyseur ou par une réaction de condensation ou par une réaction de réticulation en présence dudit peroxyde lorsqu'ils sont mis en contact les uns avec les autres.

2. Kit selon la revendication précédente, dans lequel la teneur totale en filtre solaire (A), (B), (C) et (D) est inférieure ou égale à 15 % en poids, particulièrement va de 0,01 à 10 % en poids, plus particulièrement de 0,1 à 5% en poids, par rapport au poids total de l'ensemble des compositions dans ledit kit.

3. Kit selon l'une des revendications précédentes, **caractérisé en ce que** les filtres solaires (A), (B) et (C) sont choisis parmi les dérivés de l'acide paraaminobenzoique, les dérivés salicyliques, les dérivés cinnamiques, les benzophénones et aminobenzophénones, les dérivés anthraniliques, les dérivés du dibenzoylméthane, les dérivés de β, β-diphénylacrylate, les dérivés du benzylidène camphre, les dérivés du phényl benzotriazole, les dérivés triazine, les bis-résorcinyl triazines, les dérivés d'imidazolines, les dérivés du benzalmalonate, les dérivés de 4,4-diarylbutadiène, les dérivés de benzoxazole, les mérocyanines et leurs mélanges.

4. Kit selon l'une quelconque des revendications précédentes, **caractérisé en ce que** les filtres solaires minéraux sont choisis parmi les pigments d'oxydes métalliques, enrobés ou non enrobés, et plus particulièrement les oxydes de titane, zinc, zirconium, cérium, fer ou leurs mélanges

5. Kit selon l'une quelconque des revendications précédentes, dans lequel :
- les filtres (A) sont choisis parmi Butyl Methoxydibenzoylmethane et 2-(4-diéthylamino-2-hydroxybenzoyl)-benzoate de n-hexyle ;
- les filtres (B) sont choisis parmi Ethylhexylsalicylate, Octocrylène, Ethylhexyl triazone et Ethylhexyl Methoxycinnamate ;
- les filtres (C) sont choisis parmi Drométrizole Trisiloxane, Bis-Ethylhexyloxyphénol Méthoxyphenyl Triazine ;
- les filtres (D) sont choisis parmi les oxydes de titane amorphe ou cristallisé sous forme rutile et/ou anatase.

6. Kit selon l'une quelconque des revendications précédentes, dans lequel le ratio molaire de (X)/(Y) va de 0,05 à 20 et mieux de 0,1 à 10.

7. Kit selon l'une quelconque des revendications précédentes comprenant au moins deux compositions différentes conditionnées séparément, ledit kit comprenant au moins :
- une première composition contenant, dans un milieu physiologiquement acceptable, au moins un composé (X), et
- une seconde composition contenant, dans un milieu physiologiquement acceptable, au moins un composé (Y),
ledit éventuel catalyseur ou ledit éventuel peroxyde étant présent dans l'une ou l'autre desdites première et seconde compositions ou présent éventuellement dans une troisième composition et lesdits filtres solaires (A), (B), (C) et/ou (D) étant présents dans l'une ou l'autre desdites première et seconde composition ou présent éventuellement dans ladite troisième composition.

8. Kit selon l'une quelconque des revendications précédentes, **caractérisé en ce que** les composés (X) et (Y) sont susceptibles de réagir par hydrosilylation et dans lequel le composé (X) est choisi parmi les composés siliconés comprenant au moins deux groupement aliphatiques insaturés.

9. Kit selon l'une quelconque des revendications précédentes, dans lequel le composé (X) est choisi parmi les polyorganosiloxanes comprenant des unités siloxanes de formule : 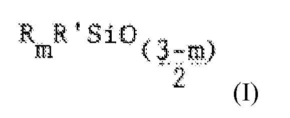 dans laquelle :
- R représente un groupe hydrocarboné monovalent, linéaire ou cyclique, comprenant de 1 à 30 atomes de carbone,
- m est égal à 1 ou 2 et
- R' représente :
• un groupement hydrocarboné aliphatique insaturé comprenant de 2 à 10, de préférence de 3 à 5 atomes de carbone ou
• un groupement hydrocarboné cyclique insaturé comprenant de 5 à 8 atomes de carbone,
dans laquelle en particulier R' représente un groupe vinyle ou un groupe -R"-CH=CHR"' dans lequel R" est une chaîne hydrocarbonée aliphatique divalente, comprenant de 1 à 8 atomes de carbone, liée à l'atome de silicium et R"' est un atome d'hydrogène ou un radical alkyle comprenant de 1 à 4 atomes de carbone, de préférence un atome d'hydrogène, et
dans laquelle encore plus particulièrement R représente un radical alkyle comprenant de 1 à 10 atomes de carbone ou encore un groupement phényle, et de préférence un radical méthyle, et R' est un groupe vinyle.

10. Kit selon l'une quelconque des revendications précédentes, dans lequel le composé (Y) est choisi parmi les organosiloxanes comprenant au moins deux groupes Si-H libres.

11. Kit selon l'une quelconque des revendications précédentes, dans lequel le composé (Y) est choisi parmi les organosiloxanes comprenant au moins une unité alkylhydrogènosiloxanes de formule suivante : 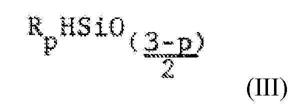 dans laquelle :
R représente un groupe hydrocarboné monovalent, linéaire ou cyclique, comprenant de 1 à 30 atomes de carbone ou un groupe phényle, et p est égal à 1 ou 2,
dans laquelle en particulier les radicaux R représentent un groupement alkyle en C₁-C₁₀, avantageusement méthyle, et
dans laquelle encore plus particulièrement les organosiloxanes Y comprennent au moins deux unités alkylhydrogènosiloxane de formule -(H₃C)(H)Si-O- et comprennent, éventuellement, des unités -(H₃C)₂SiO-.

12. Kit selon l'une quelconque des revendications précédentes, **caractérisé en ce qu'**il comprend un actif, en particulier un actif favorisant la cicatrisation et encore plus particulièrement du Madecassoside.

13. Procédé de photoprotection des matières kératiniques contre un rayonnement UV dans la gamme au-dessus de 280 nm couvrant les régions UVA et UVB, comprenant l'application sur lesdites matières:
a) d'au moins un composé (X),
b) d'au moins un composé (Y), et
c) d'au moins un système filtrant hydrophobe comprenant :
(i) au moins un filtre solaire organique hydrophobe (A) capable d'absorber les rayonnements UV de 320 à 400 nm et au moins un filtre solaire organique hydrophobe (B) capable d'absorber les rayonnements UV de 280 à 320 nm et éventuellement au moins un filtre minéral (D) ;
(ii) au moins un filtre solaire organique hydrophobe (C) capable d'absorber simultanément les rayonnements UV de 280 à 320 nm et 320 à 400 nm, et éventuellement un filtre minéral (D) ; ou
(iii) au moins un filtre solaire organique hydrophobe (A) capable d'absorber les rayonnements UV de 320 à 400 nm et au moins soit un filtre solaire organique hydrophobe (C) capable d'absorber simultanément les rayonnements UV de 280 à 320 nm et 320 à 400 nm, soit un filtre minéral (D) ;
l'un au moins desdits composés (X) et (Y) étant siliconé,
lesdits composés (X) et (Y) étant susceptibles de réagir ensemble, ladite réaction étant une réaction d'hydrosilylation en présence d'au moins un catalyseur, une réaction de condensation ou une réaction de réticulation en présence d'au moins un peroxyde à la condition que les applications a), b) et c) aient lieu soit (i) simultanément par mélange extemporané préalable, soit (ii) par mélange au moment de leur application de façon simultanée ou séquencée selon un ordre indifférent, sous réserve que les conditions dudit mélange soient propices à la réaction entre lesdits composés (X) et (Y).

14. Procédé selon la revendication 13, dans lequel la teneur totale en filtres solaires (A), (B), (C) et (D) est inférieure ou égale à 15 % en poids, particulièrement de 0,01 à 10 % en poids, plus particulièrement de 0,1 à 5 % en poids, par rapport au poids total de l'ensemble des compositions dans ledit kit.

15. Film de photoprotection susceptible d'être obtenu selon le procédé tel que défini selon la revendication 13 ou 14.

16. Composition cosmétique de photoprotection des matières kératiniques contre un rayonnement UV dans la gamme au dessus de 280 nm couvrant les UV A et les UV B comprenant, dans un milieu physiologiquement acceptable :
• au moins un composé (X),
• au moins un composé (Y),
• au moins un catalyseur, et
• au moins un système filtrant hydrophobe comprenant :
(i) au moins un filtre solaire organique hydrophobe (A) capable d'absorber les rayonnements UV de 320 à 400 nm et au moins un filtre solaire organique hydrophobe (B) capable d'absorber les rayonnements UV de 280 à 320 nm et éventuellement au moins un filtre minéral (D) ;
(ii) au moins un filtre solaire organique hydrophobe (C) capable d'absorber simultanément les rayonnements UV de 280 à 320 nm et 320 à 400 nm, et éventuellement un filtre minéral (D) ; ou
(iii) au moins un filtre solaire organique hydrophobe (A) capable d'absorber les rayonnements UV de 320 à 400 nm et au moins soit un filtre solaire organique hydrophobe (C) capable d'absorber simultanément les rayonnements UV de 280 à 320 nm et 320 à 400 nm, soit un filtre minéral (D) ;
avec au moins un des composés (X) ou (Y) étant un polyorganosiloxane, et lesdits composés (X) et (Y) étant susceptibles de réagir ensemble par une réaction d'hydrosilylation en présence dudit catalyseur, avec au moins un composé parmi les composés (X) et (Y) étant présent dans ladite composition sous une forme encapsulée, ledit catalyseur étant associé à au moins l'un desdits composés (X) ou (Y) encapsulé.
